(19) Europäisches Patentamt
European Patent Office
Office européen des brevets

(11) **EP 3 932 430 A1**

(12) **EUROPEAN PATENT APPLICATION**
published in accordance with Art. 153(4) EPC

(43) Date of publication:
**05.01.2022 Bulletin 2022/01**

(51) Int Cl.:
*A61K 47/64* (2017.01)        *A61K 38/17* (2006.01)
*A61K 9/00* (2006.01)        *A61P 19/00* (2006.01)

(21) Application number: **20762973.4**

(22) Date of filing: **27.02.2020**

(86) International application number:
**PCT/KR2020/002825**

(87) International publication number:
**WO 2020/175935 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.02.2019 KR 20190023376**

(71) Applicant: **Daewoong Pharmaceutical Co., Ltd.**
**Hwaseong-si**
**Gyenoggi-do 18623 (KR)**

(72) Inventors:
• **KOH, Jung Min**
  **Seoul 06291 (KR)**
• **KIM, Sung Sub**
  **Daejeon 34049 (KR)**
• **AHN, Kyong Hoon**
  **Seoul 06978 (KR)**

(74) Representative: **Treeby, Philip David William et al**
**Maucher Jenkins**
**26 Caxton Street**
**London SW1H 0RJ (GB)**

(54) **ALBUMIN-BOUND COMPOSITION INCLUDING LRRD2 OF SLIT3 PROTEIN FOR PREVENTION OR TREATMENT OF BONE-RELATED DISEASES**

(57)     The present invention relates to an albumin-bound composition including LRRD2 of SLIT3 protein for prevention or treatment of bone-related diseases.

[FIG. 6]

SEQUENCE LISTING

<110>  DAEWOONG PHARMACEUTICAL CO., LTD.

<120>  Composition for preventing or treating bone related disorder
       comprising HSA-conjugated Slit3 LRRD2

<130>  1067121

<150>  KR 10-2019-0023376
<151>  2019-02-27

<160>  18

<170>  PatentIn version 3.2

<210>  1
<211>  20
<212>  PRT
<213>  Artificial

<220>
<223>  SP Cystatin S

<400>  1

Met Ala Arg Pro Leu Cys Thr Leu Leu Leu Leu Met Ala Thr Leu Ala
1               5                   10                  15


Gly Ala Leu Ala
            20


<210>  2
<211>  585
<212>  PRT
<213>  Artificial

<220>
<223>  HSA

<400>  2

Asp Ala His Lys Ser Glu Val Ala His Arg Phe Lys Asp Leu Gly Glu
1               5                   10                  15


Glu Asn Phe Lys Ala Leu Val Leu Ile Ala Phe Ala Gln Tyr Leu Gln
            20                  25                  30


Gln Cys Pro Phe Glu Asp His Val Lys Leu Val Asn Glu Val Thr Glu
        35                  40                  45


Phe Ala Lys Thr Cys Val Ala Asp Glu Ser Ala Glu Asn Cys Asp Lys
    50                  55                  60


Ser Leu His Thr Leu Phe Gly Asp Lys Leu Cys Thr Val Ala Thr Leu
65                  70                  75                  80


26

Arg Glu Thr Tyr Gly Glu Met Ala Asp Cys Cys Ala Lys Gln Glu Pro
                85              90              95

Glu Arg Asn Glu Cys Phe Leu Gln His Lys Asp Asp Asn Pro Asn Leu
            100             105             110

Pro Arg Leu Val Arg Pro Glu Val Asp Val Met Cys Thr Ala Phe His
            115             120             125

Asp Asn Glu Glu Thr Phe Leu Lys Lys Tyr Leu Tyr Glu Ile Ala Arg
    130             135             140

Arg His Pro Tyr Phe Tyr Ala Pro Glu Leu Leu Phe Phe Ala Lys Arg
145             150             155             160

Tyr Lys Ala Ala Phe Thr Glu Cys Cys Gln Ala Ala Asp Lys Ala Ala
            165             170             175

Cys Leu Leu Pro Lys Leu Asp Glu Leu Arg Asp Glu Gly Lys Ala Ser
            180             185             190

Ser Ala Lys Gln Arg Leu Lys Cys Ala Ser Leu Gln Lys Phe Gly Glu
            195             200             205

Arg Ala Phe Lys Ala Trp Ala Val Ala Arg Leu Ser Gln Arg Phe Pro
    210             215             220

Lys Ala Glu Phe Ala Glu Val Ser Lys Leu Val Thr Asp Leu Thr Lys
225             230             235             240

Val His Thr Glu Cys Cys His Gly Asp Leu Leu Glu Cys Ala Asp Asp
            245             250             255

Arg Ala Asp Leu Ala Lys Tyr Ile Cys Glu Asn Gln Asp Ser Ile Ser
    260             265             270

Ser Lys Leu Lys Glu Cys Cys Glu Lys Pro Leu Leu Glu Lys Ser His
    275             280             285

Cys Ile Ala Glu Val Glu Asn Asp Glu Met Pro Ala Asp Leu Pro Ser
    290             295             300

Leu Ala Ala Asp Phe Val Glu Ser Lys Asp Val Cys Lys Asn Tyr Ala
305             310             315             320

Glu Ala Lys Asp Val Phe Leu Gly Met Phe Leu Tyr Glu Tyr Ala Arg
            325             330             335

Arg His Pro Asp Tyr Ser Val Val Leu Leu Leu Arg Leu Ala Lys Thr
                340                 345                 350

Tyr Glu Thr Thr Leu Glu Lys Cys Cys Ala Ala Ala Asp Pro His Glu
                355                 360                 365

Cys Tyr Ala Lys Val Phe Asp Glu Phe Lys Pro Leu Val Glu Glu Pro
                370                 375                 380

Gln Asn Leu Ile Lys Gln Asn Cys Glu Leu Phe Glu Gln Leu Gly Glu
385                 390                 395                 400

Tyr Lys Phe Gln Asn Ala Leu Leu Val Arg Tyr Thr Lys Lys Val Pro
                405                 410                 415

Gln Val Ser Thr Pro Thr Leu Val Glu Val Ser Arg Asn Leu Gly Lys
                420                 425                 430

Val Gly Ser Lys Cys Cys Lys His Pro Glu Ala Lys Arg Met Pro Cys
                435                 440                 445

Ala Glu Asp Tyr Leu Ser Val Val Leu Asn Gln Leu Cys Val Leu His
                450                 455                 460

Glu Lys Thr Pro Val Ser Asp Arg Val Thr Lys Cys Cys Thr Glu Ser
465                 470                 475                 480

Leu Val Asn Arg Arg Pro Cys Phe Ser Ala Leu Glu Val Asp Glu Thr
                485                 490                 495

Tyr Val Pro Lys Glu Phe Asn Ala Glu Thr Phe Thr Phe His Ala Asp
                500                 505                 510

Ile Cys Thr Leu Ser Glu Lys Glu Arg Gln Ile Lys Lys Gln Thr Ala
                515                 520                 525

Leu Val Glu Leu Val Lys His Lys Pro Lys Ala Thr Lys Glu Gln Leu
                530                 535                 540

Lys Ala Val Met Asp Asp Phe Ala Ala Phe Val Glu Lys Cys Cys Lys
545                 550                 555                 560

Ala Asp Asp Lys Glu Thr Cys Phe Ala Glu Glu Gly Lys Lys Leu Val
                565                 570                 575

Ala Ala Ser Gln Ala Ala Leu Gly Leu

580                                    585


<210>    3
<211>    209
<212>    PRT
<213>    Artificial

<220>
<223>    Slit3 LRRD2

<400>    3

Ile Ser Cys Pro Ser Pro Cys Thr Cys Ser Asn Asn Ile Val Asp Cys
1                5                   10                  15


Arg Gly Lys Gly Leu Met Glu Ile Pro Ala Asn Leu Pro Glu Gly Ile
            20                  25                  30


Val Glu Ile Arg Leu Glu Gln Asn Ser Ile Lys Ala Ile Pro Ala Gly
        35                  40                  45


Ala Phe Thr Gln Tyr Lys Lys Leu Lys Arg Ile Asp Ile Ser Lys Asn
    50                  55                  60


Gln Ile Ser Asp Ile Ala Pro Asp Ala Phe Gln Gly Leu Lys Ser Leu
65                  70                  75                  80


Thr Ser Leu Val Leu Tyr Gly Asn Lys Ile Thr Glu Ile Ala Lys Gly
            85                  90                  95


Leu Phe Asp Gly Leu Val Ser Leu Gln Leu Leu Leu Leu Asn Ala Asn
            100                 105                 110


Lys Ile Asn Cys Leu Arg Val Asn Thr Phe Gln Asp Leu Gln Asn Leu
        115                 120                 125


Asn Leu Leu Ser Leu Tyr Asp Asn Lys Leu Gln Thr Ile Ser Lys Gly
    130                 135                 140


Leu Phe Ala Pro Leu Gln Ser Ile Gln Thr Leu His Leu Ala Gln Asn
145                 150                 155                 160


Pro Phe Val Cys Asp Cys His Leu Lys Trp Leu Ala Asp Tyr Leu Gln
            165                 170                 175


Asp Asn Pro Ile Glu Thr Ser Gly Ala Arg Cys Ser Ser Pro Arg Arg
            180                 185                 190


Leu Ala Asn Lys Arg Ile Ser Gln Ile Lys Ser Lys Lys Phe Arg Cys
        195                 200                 205

Ser

<210>    4
<211>    8
<212>    PRT
<213>    Artificial

<220>
<223>    FLAG

<400>    4

Asp Tyr Lys Asp Asp Asp Asp Lys
1                   5

<210>    5
<211>    5
<212>    PRT
<213>    Artificial

<220>
<223>    GS linker

<220>
<221>    REPEAT
<222>    (1)..(5)
<223>    The amino acid residues at Position 1 to Postion 5 are repeated 1
         to 10 times.

<400>    5

Gly Gly Gly Gly Ser
1                   5

<210>    6
<211>    15
<212>    PRT
<213>    Artificial

<220>
<223>    GS linker

<400>    6

Gly Gly Gly Gly Ser Gly Gly Gly Gly Ser Gly Gly Gly Gly Ser
1                   5                   10                  15

<210>    7
<211>    681
<212>    PRT
<213>    Artificial

<220>
<223>    LRRD2-1

<400> 7

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
            20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
        50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
            100                 105                 110

Lys Gln Glu Pro Glu Arg Asn Glu Cys Phe Leu Gln His Lys Asp Asp
        115                 120                 125

Asn Pro Asn Leu Pro Arg Leu Val Arg Pro Glu Val Asp Val Met Cys
    130                 135                 140

Thr Ala Phe His Asp Asn Glu Glu Thr Phe Leu Lys Lys Tyr Leu Tyr
145                 150                 155                 160

Glu Ile Ala Arg Arg His Pro Tyr Phe Tyr Ala Pro Glu Leu Leu Phe
                165                 170                 175

Phe Ala Lys Arg Tyr Lys Ala Ala Phe Thr Glu Cys Cys Gln Ala Ala
            180                 185                 190

Asp Lys Ala Ala Cys Leu Leu Pro Lys Leu Asp Glu Leu Arg Asp Glu
        195                 200                 205

Gly Lys Ala Ser Ser Ala Lys Gln Arg Leu Lys Cys Ala Ser Leu Gln
    210                 215                 220

Lys Phe Gly Glu Arg Ala Phe Lys Ala Trp Ala Val Ala Arg Leu Ser
225                 230                 235                 240

Gln Arg Phe Pro Lys Ala Glu Phe Ala Glu Val Ser Lys Leu Val Thr

```
                    245                    250                         255


        Asp Leu Thr Lys Val His Thr Glu Cys Cys His Gly Asp Leu Leu Glu
                    260                    265                 270


        Cys Ala Asp Asp Arg Ala Asp Leu Ala Lys Tyr Ile Cys Glu Asn Gln
                    275                    280                 285


        Asp Ser Ile Ser Ser Lys Leu Lys Glu Cys Cys Glu Lys Pro Leu Leu
                290                    295                 300


        Glu Lys Ser His Cys Ile Ala Glu Val Glu Asn Asp Glu Met Pro Ala
        305                    310                315                 320


        Asp Leu Pro Ser Leu Ala Ala Asp Phe Val Glu Ser Lys Asp Val Cys
                        325                    330                 335


        Lys Asn Tyr Ala Glu Ala Lys Asp Val Phe Leu Gly Met Phe Leu Tyr
                    340                    345                 350


        Glu Tyr Ala Arg Arg His Pro Asp Tyr Ser Val Val Leu Leu Leu Arg
                    355                    360                 365


        Leu Ala Lys Thr Tyr Glu Thr Thr Leu Glu Lys Cys Cys Ala Ala Ala
            370                    375                 380


        Asp Pro His Glu Cys Tyr Ala Lys Val Phe Asp Glu Phe Lys Pro Leu
        385                    390                395                 400


        Val Glu Glu Pro Gln Asn Leu Ile Lys Gln Asn Cys Glu Leu Phe Glu
                        405                    410                 415


        Gln Leu Gly Glu Tyr Lys Phe Gln Asn Ala Leu Leu Val Arg Tyr Thr
                    420                    425                 430


        Lys Lys Val Pro Gln Val Ser Thr Pro Thr Leu Val Glu Val Ser Arg
                    435                    440                 445


        Asn Leu Gly Lys Val Gly Ser Lys Cys Cys Lys His Pro Glu Ala Lys
            450                    455                 460


        Arg Met Pro Cys Ala Glu Asp Tyr Leu Ser Val Val Leu Asn Gln Leu
        465                    470                475                 480


        Cys Val Leu His Glu Lys Thr Pro Val Ser Asp Arg Val Thr Lys Cys
                    485                    490                 495
```

```
Cys Thr Glu Ser Leu Val Asn Arg Arg Pro Cys Phe Ser Ala Leu Glu
            500                 505             510


Val Asp Glu Thr Tyr Val Pro Lys Glu Phe Asn Ala Glu Thr Phe Thr
            515                 520             525


Phe His Ala Asp Ile Cys Thr Leu Ser Glu Lys Glu Arg Gln Ile Lys
            530                 535             540


Lys Gln Thr Ala Leu Val Glu Leu Val Lys His Lys Pro Lys Ala Thr
545                 550                 555                 560


Lys Glu Gln Leu Lys Ala Val Met Asp Asp Phe Ala Ala Phe Val Glu
                565                 570                 575


Lys Cys Cys Lys Ala Asp Asp Lys Glu Thr Cys Phe Ala Glu Glu Gly
            580                 585                 590


Lys Lys Leu Val Ala Ala Ser Gln Ala Ala Leu Gly Leu Leu Thr Ser
            595                 600                 605


Leu Val Leu Tyr Gly Asn Lys Ile Thr Glu Ile Ala Lys Gly Leu Phe
            610                 615                 620


Asp Gly Leu Val Ser Leu Gln Leu Leu Leu Asn Ala Asn Lys Ile
625                 630                 635                 640


Asn Cys Leu Arg Val Asn Thr Phe Gln Asp Leu Gln Asn Leu Asn Leu
            645                 650                 655


Leu Ser Leu Tyr Asp Asn Lys Leu Gln Thr Ile Ser Lys Gly Leu Phe
            660                 665                 670


Ala Asp Tyr Lys Asp Asp Asp Lys
            675                 680


<210>   8
<211>   743
<212>   PRT
<213>   Artificial

<220>
<223>   LRRD2-2

<400>   8

Met Ala Arg Pro Leu Cys Thr Leu Leu Leu Leu Met Ala Thr Leu Ala
1                   5                   10                  15


Gly Ala Leu Ala Asp Ala His Lys Ser Glu Val Ala His Arg Phe Lys
```

```
               20                        25                         30

     Asp Leu Gly Glu Glu Asn Phe Lys Ala Leu Val Leu Ile Ala Phe Ala
             35              40              45

     Gln Tyr Leu Gln Gln Cys Pro Phe Glu Asp His Val Lys Leu Val Asn
             50              55              60

     Glu Val Thr Glu Phe Ala Lys Thr Cys Val Ala Asp Glu Ser Ala Glu
     65              70              75              80

     Asn Cys Asp Lys Ser Leu His Thr Leu Phe Gly Asp Lys Leu Cys Thr
                 85              90              95

     Val Ala Thr Leu Arg Glu Thr Tyr Gly Glu Met Ala Asp Cys Cys Ala
                 100             105             110

     Lys Gln Glu Pro Glu Arg Asn Glu Cys Phe Leu Gln His Lys Asp Asp
             115             120             125

     Asn Pro Asn Leu Pro Arg Leu Val Arg Pro Glu Val Asp Val Met Cys
         130             135             140

     Thr Ala Phe His Asp Asn Glu Glu Thr Phe Leu Lys Lys Tyr Leu Tyr
     145             150             155             160

     Glu Ile Ala Arg Arg His Pro Tyr Phe Tyr Ala Pro Glu Leu Leu Phe
                 165             170             175

     Phe Ala Lys Arg Tyr Lys Ala Ala Phe Thr Glu Cys Cys Gln Ala Ala
                 180             185             190

     Asp Lys Ala Ala Cys Leu Leu Pro Lys Leu Asp Glu Leu Arg Asp Glu
                 195             200             205

     Gly Lys Ala Ser Ser Ala Lys Gln Arg Leu Lys Cys Ala Ser Leu Gln
         210             215             220

     Lys Phe Gly Glu Arg Ala Phe Lys Ala Trp Ala Val Ala Arg Leu Ser
     225             230             235             240

     Gln Arg Phe Pro Lys Ala Glu Phe Ala Glu Val Ser Lys Leu Val Thr
                 245             250             255

     Asp Leu Thr Lys Val His Thr Glu Cys Cys His Gly Asp Leu Leu Glu
                 260             265             270
```

34

```
Cys Ala Asp Asp Arg Ala Asp Leu Ala Lys Tyr Ile Cys Glu Asn Gln
        275             280             285

Asp Ser Ile Ser Ser Lys Leu Lys Glu Cys Cys Glu Lys Pro Leu Leu
        290             295             300

Glu Lys Ser His Cys Ile Ala Glu Val Glu Asn Asp Glu Met Pro Ala
305             310             315             320

Asp Leu Pro Ser Leu Ala Ala Asp Phe Val Glu Ser Lys Asp Val Cys
            325             330             335

Lys Asn Tyr Ala Glu Ala Lys Asp Val Phe Leu Gly Met Phe Leu Tyr
        340             345             350

Glu Tyr Ala Arg Arg His Pro Asp Tyr Ser Val Val Leu Leu Leu Arg
        355             360             365

Leu Ala Lys Thr Tyr Glu Thr Thr Leu Glu Lys Cys Cys Ala Ala Ala
        370             375             380

Asp Pro His Glu Cys Tyr Ala Lys Val Phe Asp Glu Phe Lys Pro Leu
385             390             395             400

Val Glu Glu Pro Gln Asn Leu Ile Lys Gln Asn Cys Glu Leu Phe Glu
            405             410             415

Gln Leu Gly Glu Tyr Lys Phe Gln Asn Ala Leu Leu Val Arg Tyr Thr
            420             425             430

Lys Lys Val Pro Gln Val Ser Thr Pro Thr Leu Val Glu Val Ser Arg
        435             440             445

Asn Leu Gly Lys Val Gly Ser Lys Cys Cys Lys His Pro Glu Ala Lys
        450             455             460

Arg Met Pro Cys Ala Glu Asp Tyr Leu Ser Val Val Leu Asn Gln Leu
465             470             475             480

Cys Val Leu His Glu Lys Thr Pro Val Ser Asp Arg Val Thr Lys Cys
            485             490             495

Cys Thr Glu Ser Leu Val Asn Arg Arg Pro Cys Phe Ser Ala Leu Glu
            500             505             510

Val Asp Glu Thr Tyr Val Pro Lys Glu Phe Asn Ala Glu Thr Phe Thr
            515             520             525
```

```
Phe His Ala Asp Ile Cys Thr Leu Ser Glu Lys Glu Arg Gln Ile Lys
    530             535             540

Lys Gln Thr Ala Leu Val Glu Leu Val Lys His Lys Pro Lys Ala Thr
    545             550             555             560

Lys Glu Gln Leu Lys Ala Val Met Asp Asp Phe Ala Ala Phe Val Glu
                565             570             575

Lys Cys Cys Lys Ala Asp Asp Lys Glu Thr Cys Phe Ala Glu Glu Gly
            580             585             590

Lys Lys Leu Val Ala Ala Ser Gln Ala Ala Leu Gly Leu Ile Val Glu
        595             600             605

Ile Arg Leu Glu Gln Asn Ser Ile Lys Ala Ile Pro Ala Gly Ala Phe
    610             615             620

Thr Gln Tyr Lys Lys Leu Lys Arg Ile Asp Ile Ser Lys Asn Gln Ile
625             630             635             640

Ser Asp Ile Ala Pro Asp Ala Phe Gln Gly Leu Lys Ser Leu Thr Ser
                645             650             655

Leu Val Leu Tyr Gly Asn Lys Ile Thr Glu Ile Ala Lys Gly Leu Phe
            660             665             670

Asp Gly Leu Val Ser Leu Gln Leu Leu Leu Leu Asn Ala Asn Lys Ile
            675             680             685

Asn Cys Leu Arg Val Asn Thr Phe Gln Asp Leu Gln Asn Leu Asn Leu
    690             695             700

Leu Ser Leu Tyr Asp Asn Lys Leu Gln Thr Ile Ser Lys Gly Leu Phe
705             710             715             720

Ala Pro Leu Gln Ser Ile Gln Thr Leu His Leu Ala Gln Asn Pro Asp
                725             730             735

Tyr Lys Asp Asp Asp Asp Lys
                740


<210>  9
<211>  822
<212>  PRT
<213>  Artificial

<220>
```

<223> LRRD2-3

<400> 9

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
            20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
        50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
            100                 105                 110

Lys Gln Glu Pro Glu Arg Asn Glu Cys Phe Leu Gln His Lys Asp Asp
        115                 120                 125

Asn Pro Asn Leu Pro Arg Leu Val Arg Pro Glu Val Asp Val Met Cys
        130                 135                 140

Thr Ala Phe His Asp Asn Glu Glu Thr Phe Leu Lys Lys Tyr Leu Tyr
145                 150                 155                 160

Glu Ile Ala Arg Arg His Pro Tyr Phe Tyr Ala Pro Glu Leu Leu Phe
                165                 170                 175

Phe Ala Lys Arg Tyr Lys Ala Ala Phe Thr Glu Cys Cys Gln Ala Ala
            180                 185                 190

Asp Lys Ala Ala Cys Leu Leu Pro Lys Leu Asp Glu Leu Arg Asp Glu
        195                 200                 205

Gly Lys Ala Ser Ser Ala Lys Gln Arg Leu Lys Cys Ala Ser Leu Gln
        210                 215                 220

Lys Phe Gly Glu Arg Ala Phe Lys Ala Trp Ala Val Ala Arg Leu Ser
225                 230                 235                 240

```
Gln Arg Phe Pro Lys Ala Glu Phe Ala Glu Val Ser Lys Leu Val Thr
                245             250             255

Asp Leu Thr Lys Val His Thr Glu Cys Cys His Gly Asp Leu Leu Glu
                260             265             270

Cys Ala Asp Asp Arg Ala Asp Leu Ala Lys Tyr Ile Cys Glu Asn Gln
            275             280             285

Asp Ser Ile Ser Ser Lys Leu Lys Glu Cys Cys Glu Lys Pro Leu Leu
    290             295             300

Glu Lys Ser His Cys Ile Ala Glu Val Glu Asn Asp Glu Met Pro Ala
305             310             315             320

Asp Leu Pro Ser Leu Ala Ala Asp Phe Val Glu Ser Lys Asp Val Cys
                325             330             335

Lys Asn Tyr Ala Glu Ala Lys Asp Val Phe Leu Gly Met Phe Leu Tyr
            340             345             350

Glu Tyr Ala Arg Arg His Pro Asp Tyr Ser Val Val Leu Leu Leu Arg
            355             360             365

Leu Ala Lys Thr Tyr Glu Thr Thr Leu Glu Lys Cys Cys Ala Ala Ala
    370             375             380

Asp Pro His Glu Cys Tyr Ala Lys Val Phe Asp Glu Phe Lys Pro Leu
385             390             395             400

Val Glu Glu Pro Gln Asn Leu Ile Lys Gln Asn Cys Glu Leu Phe Glu
                405             410             415

Gln Leu Gly Glu Tyr Lys Phe Gln Asn Ala Leu Leu Val Arg Tyr Thr
            420             425             430

Lys Lys Val Pro Gln Val Ser Thr Pro Thr Leu Val Glu Val Ser Arg
            435             440             445

Asn Leu Gly Lys Val Gly Ser Lys Cys Cys Lys His Pro Glu Ala Lys
    450             455             460

Arg Met Pro Cys Ala Glu Asp Tyr Leu Ser Val Val Leu Asn Gln Leu
465             470             475             480

Cys Val Leu His Glu Lys Thr Pro Val Ser Asp Arg Val Thr Lys Cys
                485             490             495
```

```
Cys Thr Glu Ser Leu Val Asn Arg Arg Pro Cys Phe Ser Ala Leu Glu
        500                 505                 510

Val Asp Glu Thr Tyr Val Pro Lys Glu Phe Asn Ala Glu Thr Phe Thr
        515                 520                 525

Phe His Ala Asp Ile Cys Thr Leu Ser Glu Lys Glu Arg Gln Ile Lys
        530                 535                 540

Lys Gln Thr Ala Leu Val Glu Leu Val Lys His Lys Pro Lys Ala Thr
545                 550                 555                 560

Lys Glu Gln Leu Lys Ala Val Met Asp Asp Phe Ala Ala Phe Val Glu
                565                 570                 575

Lys Cys Cys Lys Ala Asp Asp Lys Glu Thr Cys Phe Ala Glu Glu Gly
                580                 585                 590

Lys Lys Leu Val Ala Ala Ser Gln Ala Ala Leu Gly Leu Ile Ser Cys
                595                 600                 605

Pro Ser Pro Cys Thr Cys Ser Asn Asn Ile Val Asp Cys Arg Gly Lys
        610                 615                 620

Gly Leu Met Glu Ile Pro Ala Asn Leu Pro Glu Gly Ile Val Glu Ile
625                 630                 635                 640

Arg Leu Glu Gln Asn Ser Ile Lys Ala Ile Pro Ala Gly Ala Phe Thr
                645                 650                 655

Gln Tyr Lys Lys Leu Lys Arg Ile Asp Ile Ser Lys Asn Gln Ile Ser
                660                 665                 670

Asp Ile Ala Pro Asp Ala Phe Gln Gly Leu Lys Ser Leu Thr Ser Leu
                675                 680                 685

Val Leu Tyr Gly Asn Lys Ile Thr Glu Ile Ala Lys Gly Leu Phe Asp
        690                 695                 700

Gly Leu Val Ser Leu Gln Leu Leu Leu Leu Asn Ala Asn Lys Ile Asn
705                 710                 715                 720

Cys Leu Arg Val Asn Thr Phe Gln Asp Leu Gln Asn Leu Asn Leu Leu
                725                 730                 735

Ser Leu Tyr Asp Asn Lys Leu Gln Thr Ile Ser Lys Gly Leu Phe Ala
```

                    740                         745                         750

Pro Leu Gln Ser Ile Gln Thr Leu His Leu Ala Gln Asn Pro Phe Val
        755                 760                 765

Cys Asp Cys His Leu Lys Trp Leu Ala Asp Tyr Leu Gln Asp Asn Pro
        770                 775                 780

Ile Glu Thr Ser Gly Ala Arg Cys Ser Ser Pro Arg Arg Leu Ala Asn
785                 790                 795                 800

Lys Arg Ile Ser Gln Ile Lys Ser Lys Lys Phe Arg Cys Ser Asp Tyr
                805                 810                 815

Lys Asp Asp Asp Asp Lys
            820

<210>   10
<211>   696
<212>   PRT
<213>   Artificial

<220>
<223>   LRRD2-4

<400>   10

Met Ala Arg Pro Leu Cys Thr Leu Leu Leu Leu Met Ala Thr Leu Ala
1               5                   10                  15

Gly Ala Leu Ala Asp Ala His Lys Ser Glu Val Ala His Arg Phe Lys
            20                  25                  30

Asp Leu Gly Glu Glu Asn Phe Lys Ala Leu Val Leu Ile Ala Phe Ala
            35                  40                  45

Gln Tyr Leu Gln Gln Cys Pro Phe Glu Asp His Val Lys Leu Val Asn
        50                  55                  60

Glu Val Thr Glu Phe Ala Lys Thr Cys Val Ala Asp Glu Ser Ala Glu
65                  70                  75                  80

Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
            100                 105                 110

Lys Gln Glu Pro Glu Arg Asn Glu Cys Phe Leu Gln His Lys Asp Asp
            115                 120                 125

```
Asn Pro Asn Leu Pro Arg Leu Val Arg Pro Glu Val Asp Val Met Cys
    130             135             140

Thr Ala Phe His Asp Asn Glu Glu Thr Phe Leu Lys Lys Tyr Leu Tyr
    145             150             155             160

Glu Ile Ala Arg Arg His Pro Tyr Phe Tyr Ala Pro Glu Leu Leu Phe
                165             170             175

Phe Ala Lys Arg Tyr Lys Ala Ala Phe Thr Glu Cys Cys Gln Ala Ala
            180             185             190

Asp Lys Ala Ala Cys Leu Leu Pro Lys Leu Asp Glu Leu Arg Asp Glu
        195             200             205

Gly Lys Ala Ser Ser Ala Lys Gln Arg Leu Lys Cys Ala Ser Leu Gln
    210             215             220

Lys Phe Gly Glu Arg Ala Phe Lys Ala Trp Ala Val Ala Arg Leu Ser
225             230             235             240

Gln Arg Phe Pro Lys Ala Glu Phe Ala Glu Val Ser Lys Leu Val Thr
            245             250             255

Asp Leu Thr Lys Val His Thr Glu Cys Cys His Gly Asp Leu Leu Glu
        260             265             270

Cys Ala Asp Asp Arg Ala Asp Leu Ala Lys Tyr Ile Cys Glu Asn Gln
        275             280             285

Asp Ser Ile Ser Ser Lys Leu Lys Glu Cys Cys Glu Lys Pro Leu Leu
    290             295             300

Glu Lys Ser His Cys Ile Ala Glu Val Glu Asn Asp Glu Met Pro Ala
305             310             315             320

Asp Leu Pro Ser Leu Ala Ala Asp Phe Val Glu Ser Lys Asp Val Cys
            325             330             335

Lys Asn Tyr Ala Glu Ala Lys Asp Val Phe Leu Gly Met Phe Leu Tyr
        340             345             350

Glu Tyr Ala Arg Arg His Pro Asp Tyr Ser Val Val Leu Leu Leu Arg
        355             360             365

Leu Ala Lys Thr Tyr Glu Thr Thr Leu Glu Lys Cys Cys Ala Ala Ala
```

<pre>
            370                        375                         380

    Asp Pro His Glu Cys Tyr Ala Lys Val Phe Asp Glu Phe Lys Pro Leu
    385                 390                 395                 400

    Val Glu Glu Pro Gln Asn Leu Ile Lys Gln Asn Cys Glu Leu Phe Glu
                    405                 410                 415

    Gln Leu Gly Glu Tyr Lys Phe Gln Asn Ala Leu Leu Val Arg Tyr Thr
                    420                 425                 430

    Lys Lys Val Pro Gln Val Ser Thr Pro Thr Leu Val Glu Val Ser Arg
                435                 440                 445

    Asn Leu Gly Lys Val Gly Ser Lys Cys Cys Lys His Pro Glu Ala Lys
            450                 455                 460

    Arg Met Pro Cys Ala Glu Asp Tyr Leu Ser Val Val Leu Asn Gln Leu
    465                 470                 475                 480

    Cys Val Leu His Glu Lys Thr Pro Val Ser Asp Arg Val Thr Lys Cys
                485                 490                 495

    Cys Thr Glu Ser Leu Val Asn Arg Arg Pro Cys Phe Ser Ala Leu Glu
                500                 505                 510

    Val Asp Glu Thr Tyr Val Pro Lys Glu Phe Asn Ala Glu Thr Phe Thr
                515                 520                 525

    Phe His Ala Asp Ile Cys Thr Leu Ser Glu Lys Glu Arg Gln Ile Lys
            530                 535                 540

    Lys Gln Thr Ala Leu Val Glu Leu Val Lys His Lys Pro Lys Ala Thr
    545                 550                 555                 560

    Lys Glu Gln Leu Lys Ala Val Met Asp Asp Phe Ala Ala Phe Val Glu
                565                 570                 575

    Lys Cys Cys Lys Ala Asp Asp Lys Glu Thr Cys Phe Ala Glu Glu Gly
                580                 585                 590

    Lys Lys Leu Val Ala Ala Ser Gln Ala Ala Leu Gly Leu Gly Gly Gly
                595                 600                 605

    Gly Ser Gly Gly Gly Gly Ser Gly Gly Gly Gly Ser Leu Thr Ser Leu
        610                 615                 620
</pre>

42

```
Val Leu Tyr Gly Asn Lys Ile Thr Glu Ile Ala Lys Gly Leu Phe Asp
625             630             635             640

Gly Leu Val Ser Leu Gln Leu Leu Leu Asn Ala Asn Lys Ile Asn
            645             650             655

Cys Leu Arg Val Asn Thr Phe Gln Asp Leu Gln Asn Leu Asn Leu Leu
            660             665             670

Ser Leu Tyr Asp Asn Lys Leu Gln Thr Ile Ser Lys Gly Leu Phe Ala
            675             680             685

Asp Tyr Lys Asp Asp Asp Asp Lys
    690             695
```

```
<210>  11
<211>  758
<212>  PRT
<213>  Artificial

<220>
<223>  LRRD2-5

<400>  11
```

```
Met Ala Arg Pro Leu Cys Thr Leu Leu Leu Leu Met Ala Thr Leu Ala
1               5               10              15

Gly Ala Leu Ala Asp Ala His Lys Ser Glu Val Ala His Arg Phe Lys
            20              25              30

Asp Leu Gly Glu Glu Asn Phe Lys Ala Leu Val Leu Ile Ala Phe Ala
            35              40              45

Gln Tyr Leu Gln Gln Cys Pro Phe Glu Asp His Val Lys Leu Val Asn
    50              55              60

Glu Val Thr Glu Phe Ala Lys Thr Cys Val Ala Asp Glu Ser Ala Glu
65              70              75              80

Asn Cys Asp Lys Ser Leu His Thr Leu Phe Gly Asp Lys Leu Cys Thr
            85              90              95

Val Ala Thr Leu Arg Glu Thr Tyr Gly Glu Met Ala Asp Cys Cys Ala
            100             105             110

Lys Gln Glu Pro Glu Arg Asn Glu Cys Phe Leu Gln His Lys Asp Asp
            115             120             125

Asn Pro Asn Leu Pro Arg Leu Val Arg Pro Glu Val Asp Val Met Cys
```

```
                130                      135                         140


        Thr Ala Phe His Asp Asn Glu Glu Thr Phe Leu Lys Lys Tyr Leu Tyr
        145                 150                 155                 160


        Glu Ile Ala Arg Arg His Pro Tyr Phe Tyr Ala Pro Glu Leu Leu Phe
                        165                 170                 175


        Phe Ala Lys Arg Tyr Lys Ala Ala Phe Thr Glu Cys Cys Gln Ala Ala
                    180                 185                 190


        Asp Lys Ala Ala Cys Leu Leu Pro Lys Leu Asp Glu Leu Arg Asp Glu
                    195                 200                 205


        Gly Lys Ala Ser Ser Ala Lys Gln Arg Leu Lys Cys Ala Ser Leu Gln
            210                 215                 220


        Lys Phe Gly Glu Arg Ala Phe Lys Ala Trp Ala Val Ala Arg Leu Ser
        225                 230                 235                 240


        Gln Arg Phe Pro Lys Ala Glu Phe Ala Glu Val Ser Lys Leu Val Thr
                        245                 250                 255


        Asp Leu Thr Lys Val His Thr Glu Cys Cys His Gly Asp Leu Leu Glu
                    260                 265                 270


        Cys Ala Asp Asp Arg Ala Asp Leu Ala Lys Tyr Ile Cys Glu Asn Gln
                    275                 280                 285


        Asp Ser Ile Ser Ser Lys Leu Lys Glu Cys Cys Glu Lys Pro Leu Leu
            290                 295                 300


        Glu Lys Ser His Cys Ile Ala Glu Val Glu Asn Asp Glu Met Pro Ala
        305                 310                 315                 320


        Asp Leu Pro Ser Leu Ala Ala Asp Phe Val Glu Ser Lys Asp Val Cys
                        325                 330                 335


        Lys Asn Tyr Ala Glu Ala Lys Asp Val Phe Leu Gly Met Phe Leu Tyr
                    340                 345                 350


        Glu Tyr Ala Arg Arg His Pro Asp Tyr Ser Val Val Leu Leu Leu Arg
                    355                 360                 365


        Leu Ala Lys Thr Tyr Glu Thr Thr Leu Glu Lys Cys Cys Ala Ala Ala
            370                 375                 380
```

```
Asp Pro His Glu Cys Tyr Ala Lys Val Phe Asp Glu Phe Lys Pro Leu
385             390             395             400


Val Glu Glu Pro Gln Asn Leu Ile Lys Gln Asn Cys Glu Leu Phe Glu
                405             410             415


Gln Leu Gly Glu Tyr Lys Phe Gln Asn Ala Leu Leu Val Arg Tyr Thr
            420             425             430


Lys Lys Val Pro Gln Val Ser Thr Pro Thr Leu Val Glu Val Ser Arg
        435             440             445


Asn Leu Gly Lys Val Gly Ser Lys Cys Cys Lys His Pro Glu Ala Lys
    450             455             460


Arg Met Pro Cys Ala Glu Asp Tyr Leu Ser Val Val Leu Asn Gln Leu
465             470             475             480


Cys Val Leu His Glu Lys Thr Pro Val Ser Asp Arg Val Thr Lys Cys
            485             490             495


Cys Thr Glu Ser Leu Val Asn Arg Arg Pro Cys Phe Ser Ala Leu Glu
            500             505             510


Val Asp Glu Thr Tyr Val Pro Lys Glu Phe Asn Ala Glu Thr Phe Thr
        515             520             525


Phe His Ala Asp Ile Cys Thr Leu Ser Glu Lys Glu Arg Gln Ile Lys
    530             535             540


Lys Gln Thr Ala Leu Val Glu Leu Val Lys His Lys Pro Lys Ala Thr
545             550             555             560


Lys Glu Gln Leu Lys Ala Val Met Asp Asp Phe Ala Ala Phe Val Glu
            565             570             575


Lys Cys Cys Lys Ala Asp Asp Lys Glu Thr Cys Phe Ala Glu Glu Gly
        580             585             590


Lys Lys Leu Val Ala Ala Ser Gln Ala Ala Leu Gly Leu Gly Gly Gly
        595             600             605


Gly Ser Gly Gly Gly Gly Ser Gly Gly Gly Gly Ser Ile Val Glu Ile
    610             615             620


Arg Leu Glu Gln Asn Ser Ile Lys Ala Ile Pro Ala Gly Ala Phe Thr
625             630             635             640
```

```
Gln Tyr Lys Lys Leu Lys Arg Ile Asp Ile Ser Lys Asn Gln Ile Ser
              645             650             655

Asp Ile Ala Pro Asp Ala Phe Gln Gly Leu Lys Ser Leu Thr Ser Leu
              660             665             670

Val Leu Tyr Gly Asn Lys Ile Thr Glu Ile Ala Lys Gly Leu Phe Asp
              675             680             685

Gly Leu Val Ser Leu Gln Leu Leu Leu Leu Asn Ala Asn Lys Ile Asn
      690             695             700

Cys Leu Arg Val Asn Thr Phe Gln Asp Leu Gln Asn Leu Asn Leu Leu
705             710             715             720

Ser Leu Tyr Asp Asn Lys Leu Gln Thr Ile Ser Lys Gly Leu Phe Ala
              725             730             735

Pro Leu Gln Ser Ile Gln Thr Leu His Leu Ala Gln Asn Pro Asp Tyr
              740             745             750

Lys Asp Asp Asp Asp Lys
              755
```

```
<210>  12
<211>  837
<212>  PRT
<213>  Artificial

<220>
<223>  LRRD2-6

<400>  12
```

```
Met Ala Arg Pro Leu Cys Thr Leu Leu Leu Leu Met Ala Thr Leu Ala
1               5               10              15

Gly Ala Leu Ala Asp Ala His Lys Ser Glu Val Ala His Arg Phe Lys
              20              25              30

Asp Leu Gly Glu Glu Asn Phe Lys Ala Leu Val Leu Ile Ala Phe Ala
              35              40              45

Gln Tyr Leu Gln Gln Cys Pro Phe Glu Asp His Val Lys Leu Val Asn
      50              55              60

Glu Val Thr Glu Phe Ala Lys Thr Cys Val Ala Asp Glu Ser Ala Glu
65              70              75              80
```

46

```
Asn Cys Asp Lys Ser Leu His Thr Leu Phe Gly Asp Lys Leu Cys Thr
                85                  90                  95

Val Ala Thr Leu Arg Glu Thr Tyr Gly Glu Met Ala Asp Cys Cys Ala
               100                 105                 110

Lys Gln Glu Pro Glu Arg Asn Glu Cys Phe Leu Gln His Lys Asp Asp
               115                 120                 125

Asn Pro Asn Leu Pro Arg Leu Val Arg Pro Glu Val Asp Val Met Cys
       130                 135                 140

Thr Ala Phe His Asp Asn Glu Glu Thr Phe Leu Lys Lys Tyr Leu Tyr
145                 150                 155                 160

Glu Ile Ala Arg Arg His Pro Tyr Phe Tyr Ala Pro Glu Leu Leu Phe
               165                 170                 175

Phe Ala Lys Arg Tyr Lys Ala Ala Phe Thr Glu Cys Cys Gln Ala Ala
               180                 185                 190

Asp Lys Ala Ala Cys Leu Leu Pro Lys Leu Asp Glu Leu Arg Asp Glu
       195                 200                 205

Gly Lys Ala Ser Ser Ala Lys Gln Arg Leu Lys Cys Ala Ser Leu Gln
       210                 215                 220

Lys Phe Gly Glu Arg Ala Phe Lys Ala Trp Ala Val Ala Arg Leu Ser
225                 230                 235                 240

Gln Arg Phe Pro Lys Ala Glu Phe Ala Glu Val Ser Lys Leu Val Thr
               245                 250                 255

Asp Leu Thr Lys Val His Thr Glu Cys Cys His Gly Asp Leu Leu Glu
               260                 265                 270

Cys Ala Asp Asp Arg Ala Asp Leu Ala Lys Tyr Ile Cys Glu Asn Gln
       275                 280                 285

Asp Ser Ile Ser Ser Lys Leu Lys Glu Cys Cys Glu Lys Pro Leu Leu
       290                 295                 300

Glu Lys Ser His Cys Ile Ala Glu Val Glu Asn Asp Glu Met Pro Ala
305                 310                 315                 320

Asp Leu Pro Ser Leu Ala Ala Asp Phe Val Glu Ser Lys Asp Val Cys
               325                 330                 335
```

```
Lys Asn Tyr Ala Glu Ala Lys Asp Val Phe Leu Gly Met Phe Leu Tyr
        340             345                 350

Glu Tyr Ala Arg Arg His Pro Asp Tyr Ser Val Val Leu Leu Leu Arg
        355             360                 365

Leu Ala Lys Thr Tyr Glu Thr Thr Leu Glu Lys Cys Cys Ala Ala Ala
        370             375                 380

Asp Pro His Glu Cys Tyr Ala Lys Val Phe Asp Glu Phe Lys Pro Leu
385             390             395                 400

Val Glu Glu Pro Gln Asn Leu Ile Lys Gln Asn Cys Glu Leu Phe Glu
        405             410                 415

Gln Leu Gly Glu Tyr Lys Phe Gln Asn Ala Leu Leu Val Arg Tyr Thr
        420             425                 430

Lys Lys Val Pro Gln Val Ser Thr Pro Thr Leu Val Glu Val Ser Arg
        435             440                 445

Asn Leu Gly Lys Val Gly Ser Lys Cys Cys Lys His Pro Glu Ala Lys
        450             455                 460

Arg Met Pro Cys Ala Glu Asp Tyr Leu Ser Val Val Leu Asn Gln Leu
465             470             475                 480

Cys Val Leu His Glu Lys Thr Pro Val Ser Asp Arg Val Thr Lys Cys
                485             490                 495

Cys Thr Glu Ser Leu Val Asn Arg Arg Pro Cys Phe Ser Ala Leu Glu
                500             505                 510

Val Asp Glu Thr Tyr Val Pro Lys Glu Phe Asn Ala Glu Thr Phe Thr
                515             520                 525

Phe His Ala Asp Ile Cys Thr Leu Ser Glu Lys Glu Arg Gln Ile Lys
        530             535                 540

Lys Gln Thr Ala Leu Val Glu Leu Val Lys His Lys Pro Lys Ala Thr
545             550             555                 560

Lys Glu Gln Leu Lys Ala Val Met Asp Asp Phe Ala Ala Phe Val Glu
                565             570                 575

Lys Cys Cys Lys Ala Asp Asp Lys Glu Thr Cys Phe Ala Glu Glu Gly
                580             585                 590
```

```
Lys Lys Leu Val Ala Ala Ser Gln Ala Ala Leu Gly Leu Gly Gly Gly
        595             600             605

Gly Ser Gly Gly Gly Gly Ser Gly Gly Gly Gly Ser Ile Ser Cys Pro
    610             615             620

Ser Pro Cys Thr Cys Ser Asn Asn Ile Val Asp Cys Arg Gly Lys Gly
625             630             635             640

Leu Met Glu Ile Pro Ala Asn Leu Pro Glu Gly Ile Val Glu Ile Arg
            645             650             655

Leu Glu Gln Asn Ser Ile Lys Ala Ile Pro Ala Gly Ala Phe Thr Gln
        660             665             670

Tyr Lys Lys Leu Lys Arg Ile Asp Ile Ser Lys Asn Gln Ile Ser Asp
    675             680             685

Ile Ala Pro Asp Ala Phe Gln Gly Leu Lys Ser Leu Thr Ser Leu Val
690             695             700

Leu Tyr Gly Asn Lys Ile Thr Glu Ile Ala Lys Gly Leu Phe Asp Gly
705             710             715             720

Leu Val Ser Leu Gln Leu Leu Leu Leu Asn Ala Asn Lys Ile Asn Cys
            725             730             735

Leu Arg Val Asn Thr Phe Gln Asp Leu Gln Asn Leu Asn Leu Leu Ser
        740             745             750

Leu Tyr Asp Asn Lys Leu Gln Thr Ile Ser Lys Gly Leu Phe Ala Pro
    755             760             765

Leu Gln Ser Ile Gln Thr Leu His Leu Ala Gln Asn Pro Phe Val Cys
770             775             780

Asp Cys His Leu Lys Trp Leu Ala Asp Tyr Leu Gln Asp Asn Pro Ile
785             790             795             800

Glu Thr Ser Gly Ala Arg Cys Ser Ser Pro Arg Arg Leu Ala Asn Lys
            805             810             815

Arg Ile Ser Gln Ile Lys Ser Lys Lys Phe Arg Cys Ser Asp Tyr Lys
        820             825             830

Asp Asp Asp Asp Lys
```

835

<210> 13
<211> 696
<212> PRT
<213> Artificial

<220>
<223> LRRD2-7

<400> 13

Met Ala Arg Pro Leu Cys Thr Leu Leu Leu Leu Met Ala Thr Leu Ala
1               5                   10                  15

Gly Ala Leu Ala Leu Thr Ser Leu Val Leu Tyr Gly Asn Lys Ile Thr
            20                  25                  30

Glu Ile Ala Lys Gly Leu Phe Asp Gly Leu Val Ser Leu Gln Leu Leu
        35                  40                  45

Leu Leu Asn Ala Asn Lys Ile Asn Cys Leu Arg Val Asn Thr Phe Gln
        50                  55                  60

Asp Leu Gln Asn Leu Asn Leu Leu Ser Leu Tyr Asp Asn Lys Leu Gln
65                  70                  75                  80

Thr Ile Ser Lys Gly Leu Phe Ala Asp Ala His Lys Ser Glu Val Ala
                85                  90                  95

His Arg Phe Lys Asp Leu Gly Glu Glu Asn Phe Lys Ala Leu Val Leu
            100                 105                 110

Ile Ala Phe Ala Gln Tyr Leu Gln Gln Cys Pro Phe Glu Asp His Val
            115                 120                 125

Lys Leu Val Asn Glu Val Thr Glu Phe Ala Lys Thr Cys Val Ala Asp
        130                 135                 140

Glu Ser Ala Glu Asn Cys Asp Lys Ser Leu His Thr Leu Phe Gly Asp
145                 150                 155                 160

Lys Leu Cys Thr Val Ala Thr Leu Arg Glu Thr Tyr Gly Glu Met Ala
                165                 170                 175

Asp Cys Cys Ala Lys Gln Glu Pro Glu Arg Asn Glu Cys Phe Leu Gln
            180                 185                 190

His Lys Asp Asp Asn Pro Asn Leu Pro Arg Leu Val Arg Pro Glu Val
            195                 200                 205

50

Asp Val Met Cys Thr Ala Phe His Asp Asn Glu Glu Thr Phe Leu Lys
210            215              220

Lys Tyr Leu Tyr Glu Ile Ala Arg Arg His Pro Tyr Phe Tyr Ala Pro
225            230              235              240

Glu Leu Leu Phe Phe Ala Lys Arg Tyr Lys Ala Ala Phe Thr Glu Cys
               245              250              255

Cys Gln Ala Ala Asp Lys Ala Ala Cys Leu Leu Pro Lys Leu Asp Glu
           260              265              270

Leu Arg Asp Glu Gly Lys Ala Ser Ser Ala Lys Gln Arg Leu Lys Cys
           275              280              285

Ala Ser Leu Gln Lys Phe Gly Glu Arg Ala Phe Lys Ala Trp Ala Val
   290              295              300

Ala Arg Leu Ser Gln Arg Phe Pro Lys Ala Glu Phe Ala Glu Val Ser
305            310              315              320

Lys Leu Val Thr Asp Leu Thr Lys Val His Thr Glu Cys Cys His Gly
           325              330              335

Asp Leu Leu Glu Cys Ala Asp Asp Arg Ala Asp Leu Ala Lys Tyr Ile
       340              345              350

Cys Glu Asn Gln Asp Ser Ile Ser Ser Lys Leu Lys Glu Cys Cys Glu
       355              360              365

Lys Pro Leu Leu Glu Lys Ser His Cys Ile Ala Glu Val Glu Asn Asp
   370              375              380

Glu Met Pro Ala Asp Leu Pro Ser Leu Ala Ala Asp Phe Val Glu Ser
385            390              395              400

Lys Asp Val Cys Lys Asn Tyr Ala Glu Ala Lys Asp Val Phe Leu Gly
           405              410              415

Met Phe Leu Tyr Glu Tyr Ala Arg Arg His Pro Asp Tyr Ser Val Val
       420              425              430

Leu Leu Leu Arg Leu Ala Lys Thr Tyr Glu Thr Thr Leu Glu Lys Cys
       435              440              445

Cys Ala Ala Ala Asp Pro His Glu Cys Tyr Ala Lys Val Phe Asp Glu

```
                 450                        455                             460


Phe Lys Pro Leu Val Glu Glu Pro Gln Asn Leu Ile Lys Gln Asn Cys
465                 470             475                 480


Glu Leu Phe Glu Gln Leu Gly Glu Tyr Lys Phe Gln Asn Ala Leu Leu
                485             490                 495


Val Arg Tyr Thr Lys Lys Val Pro Gln Val Ser Thr Pro Thr Leu Val
            500             505             510


Glu Val Ser Arg Asn Leu Gly Lys Val Gly Ser Lys Cys Cys Lys His
            515             520             525


Pro Glu Ala Lys Arg Met Pro Cys Ala Glu Asp Tyr Leu Ser Val Val
            530             535             540


Leu Asn Gln Leu Cys Val Leu His Glu Lys Thr Pro Val Ser Asp Arg
545                 550             555                 560


Val Thr Lys Cys Cys Thr Glu Ser Leu Val Asn Arg Arg Pro Cys Phe
                565             570                 575


Ser Ala Leu Glu Val Asp Glu Thr Tyr Val Pro Lys Glu Phe Asn Ala
            580             585                 590


Glu Thr Phe Thr Phe His Ala Asp Ile Cys Thr Leu Ser Glu Lys Glu
            595             600             605


Arg Gln Ile Lys Lys Gln Thr Ala Leu Val Glu Leu Val Lys His Lys
            610             615             620


Pro Lys Ala Thr Lys Glu Gln Leu Lys Ala Val Met Asp Asp Phe Ala
625                 630             635                 640


Ala Phe Val Glu Lys Cys Cys Lys Ala Asp Asp Lys Glu Thr Cys Phe
                645             650             655


Ala Glu Glu Gly Lys Lys Leu Val Ala Ala Ser Gln Ala Ala Leu Gly
            660             665             670


Leu Gly Gly Gly Gly Ser Gly Gly Gly Gly Ser Gly Gly Gly Gly Ser
            675             680             685


Asp Tyr Lys Asp Asp Asp Asp Lys
            690             695
```

```
<210>   14
<211>   758
<212>   PRT
<213>   Artificial

<220>
<223>   LRRD2-8

<400>   14


Met Ala Arg Pro Leu Cys Thr Leu Leu Leu Leu Met Ala Thr Leu Ala
1               5                   10                  15


Gly Ala Leu Ala Ile Val Glu Ile Arg Leu Glu Gln Asn Ser Ile Lys
            20                  25                  30


Ala Ile Pro Ala Gly Ala Phe Thr Gln Tyr Lys Lys Leu Lys Arg Ile
            35                  40                  45


Asp Ile Ser Lys Asn Gln Ile Ser Asp Ile Ala Pro Asp Ala Phe Gln
        50                  55                  60


Gly Leu Lys Ser Leu Thr Ser Leu Val Leu Tyr Gly Asn Lys Ile Thr
65                  70                  75                  80


Glu Ile Ala Lys Gly Leu Phe Asp Gly Leu Val Ser Leu Gln Leu Leu
                85                  90                  95


Leu Leu Asn Ala Asn Lys Ile Asn Cys Leu Arg Val Asn Thr Phe Gln
            100                 105                 110


Asp Leu Gln Asn Leu Asn Leu Leu Ser Leu Tyr Asp Asn Lys Leu Gln
            115                 120                 125


Thr Ile Ser Lys Gly Leu Phe Ala Pro Leu Gln Ser Ile Gln Thr Leu
        130                 135                 140


His Leu Ala Gln Asn Pro Asp Ala His Lys Ser Glu Val Ala His Arg
145                 150                 155                 160


Phe Lys Asp Leu Gly Glu Glu Asn Phe Lys Ala Leu Val Leu Ile Ala
                165                 170                 175


Phe Ala Gln Tyr Leu Gln Gln Cys Pro Phe Glu Asp His Val Lys Leu
            180                 185                 190


Val Asn Glu Val Thr Glu Phe Ala Lys Thr Cys Val Ala Asp Glu Ser
            195                 200                 205


Ala Glu Asn Cys Asp Lys Ser Leu His Thr Leu Phe Gly Asp Lys Leu
```

```
                210                          215                          220


        Cys Thr Val Ala Thr Leu Arg Glu Thr Tyr Gly Glu Met Ala Asp Cys
        225                 230                 235                 240


        Cys Ala Lys Gln Glu Pro Glu Arg Asn Glu Cys Phe Leu Gln His Lys
                        245                 250                 255


        Asp Asp Asn Pro Asn Leu Pro Arg Leu Val Arg Pro Glu Val Asp Val
                        260                 265                 270


        Met Cys Thr Ala Phe His Asp Asn Glu Glu Thr Phe Leu Lys Lys Tyr
                275                 280                 285


        Leu Tyr Glu Ile Ala Arg Arg His Pro Tyr Phe Tyr Ala Pro Glu Leu
                290                 295                 300


        Leu Phe Phe Ala Lys Arg Tyr Lys Ala Ala Phe Thr Glu Cys Cys Gln
        305                 310                 315                 320


        Ala Ala Asp Lys Ala Ala Cys Leu Leu Pro Lys Leu Asp Glu Leu Arg
                        325                 330                 335


        Asp Glu Gly Lys Ala Ser Ser Ala Lys Gln Arg Leu Lys Cys Ala Ser
                340                 345                 350


        Leu Gln Lys Phe Gly Glu Arg Ala Phe Lys Ala Trp Ala Val Ala Arg
                355                 360                 365


        Leu Ser Gln Arg Phe Pro Lys Ala Glu Phe Ala Glu Val Ser Lys Leu
                370                 375                 380


        Val Thr Asp Leu Thr Lys Val His Thr Glu Cys Cys His Gly Asp Leu
        385                 390                 395                 400


        Leu Glu Cys Ala Asp Asp Arg Ala Asp Leu Ala Lys Tyr Ile Cys Glu
                        405                 410                 415


        Asn Gln Asp Ser Ile Ser Ser Lys Leu Lys Glu Cys Cys Glu Lys Pro
                        420                 425                 430


        Leu Leu Glu Lys Ser His Cys Ile Ala Glu Val Glu Asn Asp Glu Met
                435                 440                 445


        Pro Ala Asp Leu Pro Ser Leu Ala Ala Asp Phe Val Glu Ser Lys Asp
                450                 455                 460
```

Val Cys Lys Asn Tyr Ala Glu Ala Lys Asp Val Phe Leu Gly Met Phe
465             470             475             480

Leu Tyr Glu Tyr Ala Arg Arg His Pro Asp Tyr Ser Val Val Leu Leu
                485             490             495

Leu Arg Leu Ala Lys Thr Tyr Glu Thr Thr Leu Glu Lys Cys Cys Ala
                500             505             510

Ala Ala Asp Pro His Glu Cys Tyr Ala Lys Val Phe Asp Glu Phe Lys
        515             520             525

Pro Leu Val Glu Glu Pro Gln Asn Leu Ile Lys Gln Asn Cys Glu Leu
        530             535             540

Phe Glu Gln Leu Gly Glu Tyr Lys Phe Gln Asn Ala Leu Leu Val Arg
545             550             555             560

Tyr Thr Lys Lys Val Pro Gln Val Ser Thr Pro Thr Leu Val Glu Val
                565             570             575

Ser Arg Asn Leu Gly Lys Val Gly Ser Lys Cys Cys Lys His Pro Glu
                580             585             590

Ala Lys Arg Met Pro Cys Ala Glu Asp Tyr Leu Ser Val Val Leu Asn
        595             600             605

Gln Leu Cys Val Leu His Glu Lys Thr Pro Val Ser Asp Arg Val Thr
        610             615             620

Lys Cys Cys Thr Glu Ser Leu Val Asn Arg Arg Pro Cys Phe Ser Ala
625             630             635             640

Leu Glu Val Asp Glu Thr Tyr Val Pro Lys Glu Phe Asn Ala Glu Thr
                645             650             655

Phe Thr Phe His Ala Asp Ile Cys Thr Leu Ser Glu Lys Glu Arg Gln
        660             665             670

Ile Lys Lys Gln Thr Ala Leu Val Glu Leu Val Lys His Lys Pro Lys
                675             680             685

Ala Thr Lys Glu Gln Leu Lys Ala Val Met Asp Asp Phe Ala Ala Phe
        690             695             700

Val Glu Lys Cys Cys Lys Ala Asp Asp Lys Glu Thr Cys Phe Ala Glu
705             710             715             720

55

```
Glu Gly Lys Lys Leu Val Ala Ala Ser Gln Ala Ala Leu Gly Leu Gly
            725             730             735

Gly Gly Gly Ser Gly Gly Gly Gly Ser Gly Gly Gly Gly Ser Asp Tyr
            740             745             750

Lys Asp Asp Asp Asp Lys
            755
```

```
<210>  15
<211>  837
<212>  PRT
<213>  Artificial

<220>
<223>  LRRD2-9

<400>  15
```

```
Met Ala Arg Pro Leu Cys Thr Leu Leu Leu Leu Met Ala Thr Leu Ala
1               5               10              15

Gly Ala Leu Ala Ile Ser Cys Pro Ser Pro Cys Thr Cys Ser Asn Asn
            20              25              30

Ile Val Asp Cys Arg Gly Lys Gly Leu Met Glu Ile Pro Ala Asn Leu
            35              40              45

Pro Glu Gly Ile Val Glu Ile Arg Leu Glu Gln Asn Ser Ile Lys Ala
    50              55              60

Ile Pro Ala Gly Ala Phe Thr Gln Tyr Lys Lys Leu Lys Arg Ile Asp
65              70              75              80

Ile Ser Lys Asn Gln Ile Ser Asp Ile Ala Pro Asp Ala Phe Gln Gly
            85              90              95

Leu Lys Ser Leu Thr Ser Leu Val Leu Tyr Gly Asn Lys Ile Thr Glu
            100             105             110

Ile Ala Lys Gly Leu Phe Asp Gly Leu Val Ser Leu Gln Leu Leu Leu
            115             120             125

Leu Asn Ala Asn Lys Ile Asn Cys Leu Arg Val Asn Thr Phe Gln Asp
    130             135             140

Leu Gln Asn Leu Asn Leu Leu Ser Leu Tyr Asp Asn Lys Leu Gln Thr
145             150             155             160
```

```
Ile Ser Lys Gly Leu Phe Ala Pro Leu Gln Ser Ile Gln Thr Leu His
            165             170             175

Leu Ala Gln Asn Pro Phe Val Cys Asp Cys His Leu Lys Trp Leu Ala
            180             185             190

Asp Tyr Leu Gln Asp Asn Pro Ile Glu Thr Ser Gly Ala Arg Cys Ser
            195             200             205

Ser Pro Arg Arg Leu Ala Asn Lys Arg Ile Ser Gln Ile Lys Ser Lys
    210             215             220

Lys Phe Arg Cys Ser Asp Ala His Lys Ser Glu Val Ala His Arg Phe
225             230             235             240

Lys Asp Leu Gly Glu Glu Asn Phe Lys Ala Leu Val Leu Ile Ala Phe
            245             250             255

Ala Gln Tyr Leu Gln Gln Cys Pro Phe Glu Asp His Val Lys Leu Val
            260             265             270

Asn Glu Val Thr Glu Phe Ala Lys Thr Cys Val Ala Asp Glu Ser Ala
            275             280             285

Glu Asn Cys Asp Lys Ser Leu His Thr Leu Phe Gly Asp Lys Leu Cys
    290             295             300

Thr Val Ala Thr Leu Arg Glu Thr Tyr Gly Glu Met Ala Asp Cys Cys
305             310             315             320

Ala Lys Gln Glu Pro Glu Arg Asn Glu Cys Phe Leu Gln His Lys Asp
            325             330             335

Asp Asn Pro Asn Leu Pro Arg Leu Val Arg Pro Glu Val Asp Val Met
            340             345             350

Cys Thr Ala Phe His Asp Asn Glu Glu Thr Phe Leu Lys Lys Tyr Leu
            355             360             365

Tyr Glu Ile Ala Arg Arg His Pro Tyr Phe Tyr Ala Pro Glu Leu Leu
            370             375             380

Phe Phe Ala Lys Arg Tyr Lys Ala Ala Phe Thr Glu Cys Cys Gln Ala
385             390             395             400

Ala Asp Lys Ala Ala Cys Leu Leu Pro Lys Leu Asp Glu Leu Arg Asp
            405             410             415
```

Glu Gly Lys Ala Ser Ser Ala Lys Gln Arg Leu Lys Cys Ala Ser Leu
        420             425                 430

Gln Lys Phe Gly Glu Arg Ala Phe Lys Ala Trp Ala Val Ala Arg Leu
        435             440                 445

Ser Gln Arg Phe Pro Lys Ala Glu Phe Ala Glu Val Ser Lys Leu Val
        450             455                 460

Thr Asp Leu Thr Lys Val His Thr Glu Cys Cys His Gly Asp Leu Leu
465             470             475                     480

Glu Cys Ala Asp Asp Arg Ala Asp Leu Ala Lys Tyr Ile Cys Glu Asn
                485             490                 495

Gln Asp Ser Ile Ser Ser Lys Leu Lys Glu Cys Cys Glu Lys Pro Leu
            500             505             510

Leu Glu Lys Ser His Cys Ile Ala Glu Val Glu Asn Asp Glu Met Pro
        515             520             525

Ala Asp Leu Pro Ser Leu Ala Ala Asp Phe Val Glu Ser Lys Asp Val
        530             535             540

Cys Lys Asn Tyr Ala Glu Ala Lys Asp Val Phe Leu Gly Met Phe Leu
545             550             555                     560

Tyr Glu Tyr Ala Arg Arg His Pro Asp Tyr Ser Val Val Leu Leu Leu
                565             570                 575

Arg Leu Ala Lys Thr Tyr Glu Thr Thr Leu Glu Lys Cys Cys Ala Ala
        580             585             590

Ala Asp Pro His Glu Cys Tyr Ala Lys Val Phe Asp Glu Phe Lys Pro
        595             600             605

Leu Val Glu Glu Pro Gln Asn Leu Ile Lys Gln Asn Cys Glu Leu Phe
        610             615             620

Glu Gln Leu Gly Glu Tyr Lys Phe Gln Asn Ala Leu Leu Val Arg Tyr
625             630             635                     640

Thr Lys Lys Val Pro Gln Val Ser Thr Pro Thr Leu Val Glu Val Ser
            645             650             655

Arg Asn Leu Gly Lys Val Gly Ser Lys Cys Cys Lys His Pro Glu Ala
        660             665             670

```
Lys Arg Met Pro Cys Ala Glu Asp Tyr Leu Ser Val Val Leu Asn Gln
        675             680             685

Leu Cys Val Leu His Glu Lys Thr Pro Val Ser Asp Arg Val Thr Lys
        690             695             700

Cys Cys Thr Glu Ser Leu Val Asn Arg Arg Pro Cys Phe Ser Ala Leu
705             710             715             720

Glu Val Asp Glu Thr Tyr Val Pro Lys Glu Phe Asn Ala Glu Thr Phe
            725             730             735

Thr Phe His Ala Asp Ile Cys Thr Leu Ser Glu Lys Glu Arg Gln Ile
        740             745             750

Lys Lys Gln Thr Ala Leu Val Glu Leu Val Lys His Lys Pro Lys Ala
        755             760             765

Thr Lys Glu Gln Leu Lys Ala Val Met Asp Asp Phe Ala Ala Phe Val
    770             775             780

Glu Lys Cys Cys Lys Ala Asp Asp Lys Glu Thr Cys Phe Ala Glu Glu
785             790             795             800

Gly Lys Lys Leu Val Ala Ala Ser Gln Ala Ala Leu Gly Leu Gly Gly
            805             810             815

Gly Gly Ser Gly Gly Gly Gly Ser Gly Gly Gly Gly Ser Asp Tyr Lys
        820             825             830

Asp Asp Asp Asp Lys
        835
```

```
<210>   16
<211>   711
<212>   PRT
<213>   Artificial

<220>
<223>   LRRD2-10

<400>   16
```

```
Met Ala Arg Pro Leu Cys Thr Leu Leu Leu Leu Met Ala Thr Leu Ala
1               5               10              15

Gly Ala Leu Ala Leu Thr Ser Leu Val Leu Tyr Gly Asn Lys Ile Thr
            20              25              30
```

```
Glu Ile Ala Lys Gly Leu Phe Asp Gly Leu Val Ser Leu Gln Leu Leu
    35                  40                  45

Leu Leu Asn Ala Asn Lys Ile Asn Cys Leu Arg Val Asn Thr Phe Gln
    50                  55                  60

Asp Leu Gln Asn Leu Asn Leu Leu Ser Leu Tyr Asp Asn Lys Leu Gln
65                  70                  75                  80

Thr Ile Ser Lys Gly Leu Phe Ala Gly Gly Gly Ser Gly Gly Gly
                85                  90                  95

Gly Ser Gly Gly Gly Gly Ser Asp Ala His Lys Ser Glu Val Ala His
            100                 105                 110

Arg Phe Lys Asp Leu Gly Glu Glu Asn Phe Lys Ala Leu Val Leu Ile
        115                 120                 125

Ala Phe Ala Gln Tyr Leu Gln Gln Cys Pro Phe Glu Asp His Val Lys
    130                 135                 140

Leu Val Asn Glu Val Thr Glu Phe Ala Lys Thr Cys Val Ala Asp Glu
145                 150                 155                 160

Ser Ala Glu Asn Cys Asp Lys Ser Leu His Thr Leu Phe Gly Asp Lys
                165                 170                 175

Leu Cys Thr Val Ala Thr Leu Arg Glu Thr Tyr Gly Glu Met Ala Asp
            180                 185                 190

Cys Cys Ala Lys Gln Glu Pro Glu Arg Asn Glu Cys Phe Leu Gln His
        195                 200                 205

Lys Asp Asp Asn Pro Asn Leu Pro Arg Leu Val Arg Pro Glu Val Asp
    210                 215                 220

Val Met Cys Thr Ala Phe His Asp Asn Glu Glu Thr Phe Leu Lys Lys
225                 230                 235                 240

Tyr Leu Tyr Glu Ile Ala Arg Arg His Pro Tyr Phe Tyr Ala Pro Glu
                245                 250                 255

Leu Leu Phe Phe Ala Lys Arg Tyr Lys Ala Ala Phe Thr Glu Cys Cys
            260                 265                 270

Gln Ala Ala Asp Lys Ala Ala Cys Leu Leu Pro Lys Leu Asp Glu Leu
    275                 280                 285
```

```
Arg Asp Glu Gly Lys Ala Ser Ser Ala Lys Gln Arg Leu Lys Cys Ala
    290             295             300

Ser Leu Gln Lys Phe Gly Glu Arg Ala Phe Lys Ala Trp Ala Val Ala
305             310             315             320

Arg Leu Ser Gln Arg Phe Pro Lys Ala Glu Phe Ala Glu Val Ser Lys
            325             330             335

Leu Val Thr Asp Leu Thr Lys Val His Thr Glu Cys Cys His Gly Asp
            340             345             350

Leu Leu Glu Cys Ala Asp Asp Arg Ala Asp Leu Ala Lys Tyr Ile Cys
        355             360             365

Glu Asn Gln Asp Ser Ile Ser Ser Lys Leu Lys Glu Cys Cys Glu Lys
    370             375             380

Pro Leu Leu Glu Lys Ser His Cys Ile Ala Glu Val Glu Asn Asp Glu
385             390             395             400

Met Pro Ala Asp Leu Pro Ser Leu Ala Ala Asp Phe Val Glu Ser Lys
            405             410             415

Asp Val Cys Lys Asn Tyr Ala Glu Ala Lys Asp Val Phe Leu Gly Met
        420             425             430

Phe Leu Tyr Glu Tyr Ala Arg Arg His Pro Asp Tyr Ser Val Val Leu
        435             440             445

Leu Leu Arg Leu Ala Lys Thr Tyr Glu Thr Thr Leu Glu Lys Cys Cys
    450             455             460

Ala Ala Ala Asp Pro His Glu Cys Tyr Ala Lys Val Phe Asp Glu Phe
465             470             475             480

Lys Pro Leu Val Glu Glu Pro Gln Asn Leu Ile Lys Gln Asn Cys Glu
            485             490             495

Leu Phe Glu Gln Leu Gly Glu Tyr Lys Phe Gln Asn Ala Leu Leu Val
            500             505             510

Arg Tyr Thr Lys Lys Val Pro Gln Val Ser Thr Pro Thr Leu Val Glu
    515             520             525

Val Ser Arg Asn Leu Gly Lys Val Gly Ser Lys Cys Cys Lys His Pro
```

|     |     |     | 530 |     |     |     |     |     | 535 |     |     |     |     | 540 |     |

Glu Ala Lys Arg Met Pro Cys Ala Glu Asp Tyr Leu Ser Val Val Leu
545                 550                 555                 560

Asn Gln Leu Cys Val Leu His Glu Lys Thr Pro Val Ser Asp Arg Val
                565                 570                 575

Thr Lys Cys Cys Thr Glu Ser Leu Val Asn Arg Arg Pro Cys Phe Ser
            580                 585                 590

Ala Leu Glu Val Asp Glu Thr Tyr Val Pro Lys Glu Phe Asn Ala Glu
        595                 600                 605

Thr Phe Thr Phe His Ala Asp Ile Cys Thr Leu Ser Glu Lys Glu Arg
        610                 615                 620

Gln Ile Lys Lys Gln Thr Ala Leu Val Glu Leu Val Lys His Lys Pro
625                 630                 635                 640

Lys Ala Thr Lys Glu Gln Leu Lys Ala Val Met Asp Asp Phe Ala Ala
            645                 650                 655

Phe Val Glu Lys Cys Cys Lys Ala Asp Asp Lys Glu Thr Cys Phe Ala
            660                 665                 670

Glu Glu Gly Lys Lys Leu Val Ala Ala Ser Gln Ala Ala Leu Gly Leu
        675                 680                 685

Gly Gly Gly Gly Ser Gly Gly Gly Gly Ser Gly Gly Gly Gly Ser Asp
        690                 695                 700

Tyr Lys Asp Asp Asp Asp Lys
705                 710

<210>  17
<211>  773
<212>  PRT
<213>  Artificial

<220>
<223>  LRRD2-11

<400>  17

Met Ala Arg Pro Leu Cys Thr Leu Leu Leu Leu Met Ala Thr Leu Ala
1               5                   10                  15

Gly Ala Leu Ala Ile Val Glu Ile Arg Leu Glu Gln Asn Ser Ile Lys
        20                  25                  30

```
Ala Ile Pro Ala Gly Ala Phe Thr Gln Tyr Lys Lys Leu Lys Arg Ile
        35              40              45

Asp Ile Ser Lys Asn Gln Ile Ser Asp Ile Ala Pro Asp Ala Phe Gln
        50              55              60

Gly Leu Lys Ser Leu Thr Ser Leu Val Leu Tyr Gly Asn Lys Ile Thr
65              70              75              80

Glu Ile Ala Lys Gly Leu Phe Asp Gly Leu Val Ser Leu Gln Leu Leu
            85              90              95

Leu Leu Asn Ala Asn Lys Ile Asn Cys Leu Arg Val Asn Thr Phe Gln
            100             105             110

Asp Leu Gln Asn Leu Asn Leu Leu Ser Leu Tyr Asp Asn Lys Leu Gln
        115             120             125

Thr Ile Ser Lys Gly Leu Phe Ala Pro Leu Gln Ser Ile Gln Thr Leu
    130             135             140

His Leu Ala Gln Asn Pro Gly Gly Gly Gly Ser Gly Gly Gly Gly Ser
145             150             155             160

Gly Gly Gly Gly Ser Asp Ala His Lys Ser Glu Val Ala His Arg Phe
            165             170             175

Lys Asp Leu Gly Glu Glu Asn Phe Lys Ala Leu Val Leu Ile Ala Phe
        180             185             190

Ala Gln Tyr Leu Gln Gln Cys Pro Phe Glu Asp His Val Lys Leu Val
    195             200             205

Asn Glu Val Thr Glu Phe Ala Lys Thr Cys Val Ala Asp Glu Ser Ala
    210             215             220

Glu Asn Cys Asp Lys Ser Leu His Thr Leu Phe Gly Asp Lys Leu Cys
225             230             235             240

Thr Val Ala Thr Leu Arg Glu Thr Tyr Gly Glu Met Ala Asp Cys Cys
            245             250             255

Ala Lys Gln Glu Pro Glu Arg Asn Glu Cys Phe Leu Gln His Lys Asp
        260             265             270

Asp Asn Pro Asn Leu Pro Arg Leu Val Arg Pro Glu Val Asp Val Met
```

|       |       |       | 275   |       |       |       |       | 280   |       |       |       |       | 285   |       |
|-------|-------|-------|-------|-------|-------|-------|-------|-------|-------|-------|-------|-------|-------|-------|

Cys Thr Ala Phe His Asp Asn Glu Glu Thr Phe Leu Lys Lys Tyr Leu
   290             295             300

Tyr Glu Ile Ala Arg Arg His Pro Tyr Phe Tyr Ala Pro Glu Leu Leu
305            310          315             320

Phe Phe Ala Lys Arg Tyr Lys Ala Ala Phe Thr Glu Cys Cys Gln Ala
         325          330          335

Ala Asp Lys Ala Ala Cys Leu Leu Pro Lys Leu Asp Glu Leu Arg Asp
       340          345          350

Glu Gly Lys Ala Ser Ser Ala Lys Gln Arg Leu Lys Cys Ala Ser Leu
       355          360          365

Gln Lys Phe Gly Glu Arg Ala Phe Lys Ala Trp Ala Val Ala Arg Leu
   370              375          380

Ser Gln Arg Phe Pro Lys Ala Glu Phe Ala Glu Val Ser Lys Leu Val
385            390          395             400

Thr Asp Leu Thr Lys Val His Thr Glu Cys Cys His Gly Asp Leu Leu
       405          410          415

Glu Cys Ala Asp Asp Arg Ala Asp Leu Ala Lys Tyr Ile Cys Glu Asn
       420          425          430

Gln Asp Ser Ile Ser Ser Lys Leu Lys Glu Cys Cys Glu Lys Pro Leu
       435          440          445

Leu Glu Lys Ser His Cys Ile Ala Glu Val Glu Asn Asp Glu Met Pro
       450          455          460

Ala Asp Leu Pro Ser Leu Ala Ala Asp Phe Val Glu Ser Lys Asp Val
465            470          475             480

Cys Lys Asn Tyr Ala Glu Ala Lys Asp Val Phe Leu Gly Met Phe Leu
       485          490          495

Tyr Glu Tyr Ala Arg Arg His Pro Asp Tyr Ser Val Val Leu Leu Leu
       500          505          510

Arg Leu Ala Lys Thr Tyr Glu Thr Thr Leu Glu Lys Cys Cys Ala Ala
       515          520          525

```
Ala Asp Pro His Glu Cys Tyr Ala Lys Val Phe Asp Glu Phe Lys Pro
    530                 535                 540

Leu Val Glu Glu Pro Gln Asn Leu Ile Lys Gln Asn Cys Glu Leu Phe
545                 550                 555                 560

Glu Gln Leu Gly Glu Tyr Lys Phe Gln Asn Ala Leu Leu Val Arg Tyr
                565                 570                 575

Thr Lys Lys Val Pro Gln Val Ser Thr Pro Thr Leu Val Glu Val Ser
            580                 585                 590

Arg Asn Leu Gly Lys Val Gly Ser Lys Cys Cys Lys His Pro Glu Ala
        595                 600                 605

Lys Arg Met Pro Cys Ala Glu Asp Tyr Leu Ser Val Val Leu Asn Gln
    610                 615                 620

Leu Cys Val Leu His Glu Lys Thr Pro Val Ser Asp Arg Val Thr Lys
625                 630                 635                 640

Cys Cys Thr Glu Ser Leu Val Asn Arg Arg Pro Cys Phe Ser Ala Leu
                645                 650                 655

Glu Val Asp Glu Thr Tyr Val Pro Lys Glu Phe Asn Ala Glu Thr Phe
            660                 665                 670

Thr Phe His Ala Asp Ile Cys Thr Leu Ser Glu Lys Glu Arg Gln Ile
        675                 680                 685

Lys Lys Gln Thr Ala Leu Val Glu Leu Val Lys His Lys Pro Lys Ala
    690                 695                 700

Thr Lys Glu Gln Leu Lys Ala Val Met Asp Asp Phe Ala Ala Phe Val
705                 710                 715                 720

Glu Lys Cys Cys Lys Ala Asp Asp Lys Glu Thr Cys Phe Ala Glu Glu
                725                 730                 735

Gly Lys Lys Leu Val Ala Ala Ser Gln Ala Ala Leu Gly Leu Gly Gly
            740                 745                 750

Gly Gly Ser Gly Gly Gly Gly Ser Gly Gly Gly Gly Ser Asp Tyr Lys
        755                 760                 765

Asp Asp Asp Asp Lys
    770
```

65

<210> 18
<211> 852
<212> PRT
<213> Artificial

<220>
<223> LRRD2-12

<400> 18

Met Ala Arg Pro Leu Cys Thr Leu Leu Leu Leu Met Ala Thr Leu Ala
1               5                   10                  15

Gly Ala Leu Ala Ile Ser Cys Pro Ser Pro Cys Thr Cys Ser Asn Asn
            20                  25                  30

Ile Val Asp Cys Arg Gly Lys Gly Leu Met Glu Ile Pro Ala Asn Leu
            35                  40                  45

Pro Glu Gly Ile Val Glu Ile Arg Leu Glu Gln Asn Ser Ile Lys Ala
        50                  55                  60

Ile Pro Ala Gly Ala Phe Thr Gln Tyr Lys Lys Leu Lys Arg Ile Asp
65                  70                  75                  80

Ile Ser Lys Asn Gln Ile Ser Asp Ile Ala Pro Asp Ala Phe Gln Gly
                85                  90                  95

Leu Lys Ser Leu Thr Ser Leu Val Leu Tyr Gly Asn Lys Ile Thr Glu
            100                 105                 110

Ile Ala Lys Gly Leu Phe Asp Gly Leu Val Ser Leu Gln Leu Leu Leu
            115                 120                 125

Leu Asn Ala Asn Lys Ile Asn Cys Leu Arg Val Asn Thr Phe Gln Asp
        130                 135                 140

Leu Gln Asn Leu Asn Leu Leu Ser Leu Tyr Asp Asn Lys Leu Gln Thr
145                 150                 155                 160

Ile Ser Lys Gly Leu Phe Ala Pro Leu Gln Ser Ile Gln Thr Leu His
                165                 170                 175

Leu Ala Gln Asn Pro Phe Val Cys Asp Cys His Leu Lys Trp Leu Ala
            180                 185                 190

Asp Tyr Leu Gln Asp Asn Pro Ile Glu Thr Ser Gly Ala Arg Cys Ser
            195                 200                 205

```
Ser Pro Arg Arg Leu Ala Asn Lys Arg Ile Ser Gln Ile Lys Ser Lys
    210                 215                 220

Lys Phe Arg Cys Ser Gly Gly Gly Gly Ser Gly Gly Gly Gly Ser Gly
    225                 230                 235                 240

Gly Gly Ser Asp Ala His Lys Ser Glu Val Ala His Arg Phe Lys
                245                 250                 255

Asp Leu Gly Glu Glu Asn Phe Lys Ala Leu Val Leu Ile Ala Phe Ala
                260                 265                 270

Gln Tyr Leu Gln Gln Cys Pro Phe Glu Asp His Val Lys Leu Val Asn
        275                 280                 285

Glu Val Thr Glu Phe Ala Lys Thr Cys Val Ala Asp Glu Ser Ala Glu
        290                 295                 300

Asn Cys Asp Lys Ser Leu His Thr Leu Phe Gly Asp Lys Leu Cys Thr
    305                 310                 315                 320

Val Ala Thr Leu Arg Glu Thr Tyr Gly Glu Met Ala Asp Cys Cys Ala
                325                 330                 335

Lys Gln Glu Pro Glu Arg Asn Glu Cys Phe Leu Gln His Lys Asp Asp
                340                 345                 350

Asn Pro Asn Leu Pro Arg Leu Val Arg Pro Glu Val Asp Val Met Cys
        355                 360                 365

Thr Ala Phe His Asp Asn Glu Glu Thr Phe Leu Lys Lys Tyr Leu Tyr
    370                 375                 380

Glu Ile Ala Arg Arg His Pro Tyr Phe Tyr Ala Pro Glu Leu Leu Phe
    385                 390                 395                 400

Phe Ala Lys Arg Tyr Lys Ala Ala Phe Thr Glu Cys Cys Gln Ala Ala
                405                 410                 415

Asp Lys Ala Ala Cys Leu Leu Pro Lys Leu Asp Glu Leu Arg Asp Glu
                420                 425                 430

Gly Lys Ala Ser Ser Ala Lys Gln Arg Leu Lys Cys Ala Ser Leu Gln
                435                 440                 445

Lys Phe Gly Glu Arg Ala Phe Lys Ala Trp Ala Val Ala Arg Leu Ser
    450                 455                 460
```

```
Gln Arg Phe Pro Lys Ala Glu Phe Ala Glu Val Ser Lys Leu Val Thr
465             470             475             480

Asp Leu Thr Lys Val His Thr Glu Cys Cys His Gly Asp Leu Leu Glu
                485             490             495

Cys Ala Asp Asp Arg Ala Asp Leu Ala Lys Tyr Ile Cys Glu Asn Gln
        500             505             510

Asp Ser Ile Ser Ser Lys Leu Lys Glu Cys Cys Glu Lys Pro Leu Leu
        515             520             525

Glu Lys Ser His Cys Ile Ala Glu Val Glu Asn Asp Glu Met Pro Ala
        530             535             540

Asp Leu Pro Ser Leu Ala Ala Asp Phe Val Glu Ser Lys Asp Val Cys
545             550             555             560

Lys Asn Tyr Ala Glu Ala Lys Asp Val Phe Leu Gly Met Phe Leu Tyr
            565             570             575

Glu Tyr Ala Arg Arg His Pro Asp Tyr Ser Val Val Leu Leu Leu Arg
            580             585             590

Leu Ala Lys Thr Tyr Glu Thr Thr Leu Glu Lys Cys Cys Ala Ala Ala
            595             600             605

Asp Pro His Glu Cys Tyr Ala Lys Val Phe Asp Glu Phe Lys Pro Leu
        610             615             620

Val Glu Glu Pro Gln Asn Leu Ile Lys Gln Asn Cys Glu Leu Phe Glu
625             630             635             640

Gln Leu Gly Glu Tyr Lys Phe Gln Asn Ala Leu Leu Val Arg Tyr Thr
            645             650             655

Lys Lys Val Pro Gln Val Ser Thr Pro Thr Leu Val Glu Val Ser Arg
            660             665             670

Asn Leu Gly Lys Val Gly Ser Lys Cys Cys Lys His Pro Glu Ala Lys
            675             680             685

Arg Met Pro Cys Ala Glu Asp Tyr Leu Ser Val Val Leu Asn Gln Leu
        690             695             700

Cys Val Leu His Glu Lys Thr Pro Val Ser Asp Arg Val Thr Lys Cys
705             710             715             720
```

```
Cys Thr Glu Ser Leu Val Asn Arg Arg Pro Cys Phe Ser Ala Leu Glu
            725             730              735

Val Asp Glu Thr Tyr Val Pro Lys Glu Phe Asn Ala Glu Thr Phe Thr
            740             745              750

Phe His Ala Asp Ile Cys Thr Leu Ser Glu Lys Glu Arg Gln Ile Lys
            755             760              765

Lys Gln Thr Ala Leu Val Glu Leu Val Lys His Lys Pro Lys Ala Thr
            770             775              780

Lys Glu Gln Leu Lys Ala Val Met Asp Asp Phe Ala Ala Phe Val Glu
785             790             795              800

Lys Cys Cys Lys Ala Asp Asp Lys Glu Thr Cys Phe Ala Glu Glu Gly
            805             810              815

Lys Lys Leu Val Ala Ala Ser Gln Ala Ala Leu Gly Leu Gly Gly Gly
            820             825              830

Gly Ser Gly Gly Gly Gly Ser Gly Gly Gly Gly Ser Asp Tyr Lys Asp
            835             840              845

Asp Asp Asp Lys
            850
```

**Description**

[Technical Field]

The present invention relates to a composition comprising albumin-bound LRRD2 of the Slit3 protein for prevention or treatment of bone-related diseases.

[Background Art]

Slit proteins are well-known proteins that regulate the movement of neurons and axons during the developmental process of the nervous system. It is known that a Slit protein can act with a Robo receptor to regulate physiological activity, and serves as a factor that regulates various intracellular processes in various tissues such as heart, lung, kidney, and breast tissues, and as it has been recently reported that Slit proteins play an important role in the regulation of growth, adhesion ability, and migration ability of cells, it was reported that Slit proteins can participate in the migration in the differentiation of cells and the occurrence and metastasis of cancer.

Based on such a background, the present inventors have revealed that LRRD2 of Slit3 can be usefully used as a composition for prevention or treatment of fractures or osteoporosis and a biomarker for predicting the risk of outbreak of fractures or osteoporosis because LRRD2 of Slit3 increases bone formation, reduces bone resorption, and has a negative correlation with the incidence of osteoporosis in cell and animal models (Korean Patent No. 10-1617497). Since LRRD2 needs to be administered as an injection, patients need to visit the hospital, but LRRD2 has a very short *in vivo* half-life, so its administration cycle should be shortened in order to exhibit the medicinal effects thereof, and it is expected that a problem in that its efficacy is reduced due to the associated excessive use of the drug occurs.

Thus, the present inventors have developed an HSA-Slit3 LRRD2 fusion protein with improved efficacy by increasing the *in vivo* half-life of LRRD2, thereby completing the present invention.

[Disclosure]

[Technical Problem]

An object of the present invention is to provide a composition in which the efficacy of LRRD2 of the Slit3 protein for preventing or treating bone-related diseases has been improved.

[Technical Solution]

To achieve the above-described object, the present invention provides a pharmaceutical composition comprising albumin-bound LRRD2 of the Slit3 protein for prevention or treatment of bone-related diseases.

According to a preferred exemplary embodiment of the present invention, the albumin may be human serum albumin.

According to another preferred exemplary embodiment of the present invention, the human serum albumin may be bound to the N-terminus of LRRD2 of the Slit3 protein.

According to still another preferred exemplary embodiment of the present invention, the human serum albumin may include an amino acid sequence of SEQ ID NO: 2.

According to yet another preferred exemplary embodiment of the present invention, the LRRD2 of the Slit3 protein may include an amino acid sequence of SEQ ID NO: 3.

According to yet another preferred exemplary embodiment of the present invention, the pharmaceutical composition may further include a linker between the albumin and the LRRD2 of the Slit3 protein.

According to yet another preferred exemplary embodiment of the present invention, the linker may be (GGGGS)n (SEQ ID NO: 5), wherein n may be an integer from 1 to 10.

According to yet another preferred exemplary embodiment of the present invention, the pharmaceutical composition may be administered as an injection.

According to yet another preferred exemplary embodiment of the present invention, the bone-related disease may be any one or more selected from the group consisting of osteoporosis, fractures, bone loss, osteoarthritis, metastatic bone cancer, and Paget's disease.

[Advantageous Effects]

Since albumin-bound LRRD2 of the Slit3 protein exhibits the same cytological efficacy as albumin-unbound LRRD2 of the Slit3 protein and has a significantly increased *in vivo* half-life compared to albumin-unbound LRRD2 of the Slit3 protein, bone-related diseases can be more effectively prevented or treated.

[Description of Drawings]

FIG. 1 illustrates a composition of a fusion protein in which an albumin of the present invention is bound to the N-terminus of Slit3 LRRD2 and an amino acid sequence thereof.

FIG. 2 illustrates the results of performing SDS-PAGE after isolating and purifying an SP cystatin S-HSA-Slit3LRRD2 fusion protein.

FIG. 3 graphically illustrates the receptor binding ability of various forms of HSA-Slit3 LRRD2 fusion proteins.

FIG. 4 illustrates the results of confirming the migration ability of osteoblasts according to the treatment of various forms of HSA-Slit3 LRRD2 fusion proteins.

FIG. 5 illustrates the results of confirming the differentiation ability of osteoclasts according to the treatment of various forms of HSA-Slit3 LRRD2 fusion proteins.

FIG. 6 illustrates the results of confirming the results of confirming (A) the migration ability of osteoblasts, (B) the β-catenin activity of osteoblasts, and (C) the differentiation ability of osteoclasts, according to the treatment of the HSA-Slit3 LRRD2 fusion protein.

FIG. 7 illustrates the plasma concentration-time profiles of Slit3 LRRD2 after IV administration of Slit3 LRRD2 (●, "Slit3") and HSA-Slit3 LRRD2 (■, "HSA-Slit3 ") to fasted male ICR mice.

[Modes of the Invention]

As described above, LRRD2 of the Slit3 protein may be used for prevention or treatment of fractures or osteoporosis by increasing bone formation and reducing bone resorption, but LRRD2 has a very short *in vivo* half-life, so its administration cycle should be shortened in order to exhibit the medicinal effects thereof, and it is expected that a problem in that its efficacy is reduced due to the associated excessive use of the drug occurs

Thus, the present inventors have sought a solution to the above-described problem by enhancing the *in vivo* half-life of LRRD2 to develop an HSA-Slit3 LRRD2 fusion protein with improved efficacy. Since albumin-bound LRRD2 of the Slit3 protein exhibits the same cytological efficacy as albumin-unbound LRRD2 of the Slit3 protein and has a significantly increased in vivo half-life compared to albumin-unbound LRRD2 of the Slit3 protein, bone-related diseases can be more effectively prevented or treated.

Hereinafter, the present invention will be described in more detail.

The present invention provides a composition comprising albumin-bound LRRD2 of the SLIT3 protein for prevention or treatment of bone-related diseases.

In the pharmaceutical composition of the present invention, the "LRRD2 of the Slit3 protein" refers to a second leucine-rich repeat domain (LRRD2) in the Slit3 protein.

As used herein, the term "Slit3 LRRD2" refers to "LRRD2 of the Slit3 protein" and may be used interchangeably.

In the pharmaceutical composition of the present invention, the albumin may be human serum albumin, rhesus serum albumin (RhSA), cynomolgus monkey serum albumin (CySA), or murine serum albumin (MuSA), and preferably human serum albumin. The Slit3 LRRD2 has an *in vivo* half-life of Slit3 LRRD2 in the presence of human serum albumin that is at least 10-fold longer than that of Slit3 LRRD2 in the absence of human serum albumin.

In a specific exemplary embodiment of the present invention, the serum half-life of Slit3 LRRD2 in the presence of human serum albumin is 14-fold longer than that of Slit3 LRRD2 in the absence of human serum albumin.

In the pharmaceutical composition of the present invention, the human serum albumin and Slit3 LRRD2 may be bound in the order of the human serum albumin and Slit3 LRRD2, or vice versa. Preferably, the human serum albumin and Slit3 LRRD2 are bound in this order. For example, when the human serum albumin binds to the N-terminus of Slit3 LRRD2, Slit3 LRRD2 has the best *in vivo* half-life and therapeutic efficacy for bone-related diseases, and when the human serum albumin binds to the C-terminus thereof, it is possible to exhibit an effective efficacy even though there may be a difference in degree.

In the pharmaceutical composition of the present invention, as the human serum albumin, a full-length amino acid sequence consisting of 609 amino acids or a fragment comprising a partial amino acid sequence thereof may be used. The full-length amino acid sequence of the human serum albumin is disclosed in the NCBI GenBank: AAA98797.1, and in an exemplary embodiment of the present invention, the form of a fragment consisting of the 25th to 609th amino acids (585 amino acids) from the full-length human serum albumin consisting of 609 amino acids was used. In the pharmaceutical composition of the present invention, the human serum albumin consists of the following SEQ ID NO: 2:

DAHKSEVAHRFKDLGEENFKALVLIAFAQYLQQCPFEDHVKL

VNEVTEFAKTCVADESAENCDKSLHTLFGDKLCTVATLRETYGEMA

DCCAKQEPERNECFLQHKDDNPNLPRLVRPEVDVMCTAFHDNEETFL

KKYLYEIARRHPYFYAPELLFFAKRYKAAFTECCQAADKAACLLPKL

DELRDEGKASSAKQRLKCASLQKFGERAFKAWAVARLSQRFPKAEF

AEVSKLVTDLTKVHTECCHGDLLECADDRADLAKYICENQDSISSKL

KECCEKPLLEKSHCIAEVENDEMPADLPSLAADFVESKDVCKNYAEAKDVF

LGMFLYEYARRHPDYSVVLLLRLAKTYETTLEKCCAAADPHECYAKVFDEF

KPLVEEPQNLIKQNCELFEQLGEYKFQNALLVRYTKKVPQVSTPTLVEVSRN

LGKVGSKCCKHPEAKRMPCAEDYLSVVLNQLCVLHEKTPVSDRVTKCCTES

LVNRRPCFSALEVDETYVPKEFNAETFTFHADICTLSEKERQIKKQTALVELV

KHKPKATKEQLKAVMDDFAAFVEKCCKADDKETCFAEEGKKLVAASQAAL

GL (SEQ ID NO: 2).

In the pharmaceutical composition of the present invention, the Slit3 LRRD2 is human-derived, and a full-length amino acid sequence of LRRD2 in the Slit3 protein consisting of 1523 amino acids or a fragment comprising a partial amino acid sequence thereof may be used. The full-length amino acid sequence of the Slit3 protein is disclosed in the NCBI GenBank: AAQ89243.1, and in an exemplary embodiment of the present invention, as Slit3 LRRD2, the form of a fragment consisting of the 278th to 486th amino acids (209 amino acids) from the full-length Slit3 protein consisting of 1523 amino acids was used. In the pharmaceutical composition of the present invention, Slit3 LRRD2 consists of an amino acid sequence of the following SEQ ID NO: 3:

ISCPSPCTCSNNIVDCRGKGLMEIPANLPEGIVEIRLEQNSIKAIPAGAF

TQYKKLKRIDISKNQISDIAPDAFQGLKSLTSLVLYGNKITEIAKGLFDGLVSL

QLLLLNANKINCLRVNTFQDLQNLNLLSLYDNKLQTISKGLFAPLQSIQTLHL

AQNPFVCDCHLKWLADYLQDNPIETSGARCSSPRRLANKRISQIKSKKFRCS

(SEQ ID NO: 3).

In the pharmaceutical composition of the present invention, "Slit3 LRRD2" may include a functional equivalent of the amino acid sequence of SEQ ID NO: 3.

The "functional equivalent" has a sequence homology of at least 70% or more, preferably 80% or more, more preferably 90% or more, and even more preferably 95% or more with the amino acid sequences of SEQ ID NOS: 1 to 4 of the present invention by the addition, substitution, or deletion of amino acids of a protein or peptide, and refers to a protein or peptide exhibiting physiological activity substantially equivalent to that of a protein or peptide consisting of amino acid sequences of SEQ ID NOS: 1 to 4.

Specifically, for the fusion protein included in the pharmaceutical composition of the present invention, not only a protein or peptide having a wild-type amino acid sequence thereof, but also an amino acid sequence variant thereof may also be included in the scope of the present invention. The amino acid sequence variant refers to a protein or peptide having a sequence different from a wild-type amino acid sequence of Slit3 LRRD2 by deletion, insertion, non-conservative or conservative substitution of one or more amino acid residues, or a combination thereof.

Amino acid exchanges possible in proteins and peptides that do not entirely change the activities of the molecules are known in the art (H. Neurath, R.L.Hill, The Proteins, Academic Press, New York, 1979). The most typically occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may also be modified by phosphorylation, sulfation, acetylation, glycosylation, methylation, farnesylation, or the like.

The Slit3 LRRD2 of the present invention, or variants thereof can be extracted from nature or synthesized (Merrifleld, J. Amer. Chem. Soc. 85:2149-2156, 85:2149-2156, 1963) or prepared by a gene recombinant method based on a DNA sequence (Sambrook et al., Molecular Cloning, Cold Spring Harbour Laboratory Press, New York, USA, 2d Ed., 1989).

In the pharmaceutical composition of the present invention, a linker may be further included between albumin and LRRD2 of the Slit3 protein. A preferred linker type may be (GGGS)n (SEQ ID NO: 5), wherein n may be an integer from 1 to 10, and preferably n may be an integer from 1 to 5.

The Slit3 LRRD2 of the present invention may be subjected not only to albumin fusion, but also to fusion or PEGylation of an Fc protein of IgG, and the like in order to enhance the *in vivo* half-life thereof.

The pharmaceutical composition of the present invention may be in the form of various oral or parenteral formulations. When the pharmaceutical composition is formulated, the composition may be prepared by using a buffer (for example, a saline solution or PBS), an antioxidant, a bacteriostatic agent, a chelating agent (for example, EDTA or glutathione), a filler, an extender, a binder, an adjuvant (for example, aluminum hydroxide), a suspension agent, a thickener, a wetting agent, a disintegrant, or a surfactant, a diluent or an excipient.

Examples of a solid preparation for oral administration include a tablet, a pill, a powder, granules, a capsule, and the like, and the solid preparation is prepared by mixing one or more compounds with one or more excipients, for example, starch (including corn starch, wheat starch, rice starch, potato starch, and the like), calcium carbonate, sucrose, lactose, dextrose, sorbitol, mannitol, xylitol, erythritol maltitol, cellulose, methyl cellulose, sodium carboxymethylcellulose and hydroxypropymethyl-cellulose, gelatin, or the like. For example, a tablet or a sugar tablet may be obtained by blending an active ingredient with a solid excipient, pulverizing the resulting blend, adding a suitable auxiliary agent thereto, and then processing the resulting mixture into a granular mixture.

Further, in addition to simple excipients, lubricants such as magnesium stearate and talc are also used. A liquid preparation for oral administration corresponds to a suspension agent, a liquid for internal use, an emulsion, a syrup, and the like, and the liquid preparation may include, in addition to water and liquid paraffin which are simple commonly used diluents, various excipients, for example, a wetting agent, a sweetener, an odorant, a preservative, and the like. In addition, in some cases, crosslinked polyvinyl pyrrolidone, agar, alginic acid, sodium alginate, or the like may be added as a disintegrant, and an anti-coagulant, a lubricant, a wetting agent, a flavoring agent, an emulsifier, an antiseptic, and the like may be additionally added.

Examples of a preparation for parenteral administration include an aqueous sterile solution, a non-aqueous solvent, a suspension solvent, an emulsion, a freeze-dried preparation, a suppository, or the like. As the non-aqueous solvent and the suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerol, gelatin, and the like.

The pharmaceutical composition of the present invention may be administered orally or parenterally, and, when administered parenterally, may be formulated in the form of a preparation for external application to the skin; an injection administered intraperitoneally, rectally, intravenously, muscularly, subcutaneously, or intracerebroventricularly, or via cervical intrathecal injection; a percutaneous administration agent; or a nasal inhaler according to a method known in the art.

The injection must be sterilized and protected from contamination of microorganisms such as bacteria and fungi. Examples of a suitable carrier for the injection may be, but are not limited to, a solvent or a dispersion medium including water, ethanol, polyols (for example, glycerol, propylene glycol, liquid polyethylene glycol, and the like), mixtures thereof, and/or vegetable oils. More preferably, as a suitable carrier, it is possible to use an isotonic solution such as Hank's solution, Ringer's solution, triethanolamine-containing phosphate buffered saline (PBS) or sterile water for injection, 10% ethanol, 40% propylene glycol, and 5% dextrose, and the like. To protect the injection from microbial contamination, various antimicrobial agents and antifungal agents such as a paraben, chlorobutanol, phenol, sorbic acid, and thimerosal may be additionally included. Furthermore, in most cases, the injection may additionally include an isotonic agent such as sugar or sodium chloride.

Examples of the percutaneous administration agent include a form such as an ointment, a cream, a lotion, a gel, a solution for external use, a paste, a liniment, and an aerosol. The transdermal administration as described above means that an effective amount of an active ingredient contained in a pharmaceutical composition is delivered into the skin via local administration thereof to the skin.

In the case of a preparation for inhalation, the fusion protein used according to the present invention may be conveniently delivered in the form of an aerosol spray from a pressurized pack or a nebulizer by using a suitable propellant, for example, dichlorofluoromethane, trichlorofluoromethane, dichlorotetrafluoroethane, carbon dioxide, or other suitable gases. In the case of the pressurized aerosol, a dosage unit may be determined by providing a valve for transferring a metered amount. For example, a gelatin capsule and a cartridge for use in an inhaler or insufflator may be formulated so as to contain a powder mixture of a compound and a suitable powder base such as lactose or starch. Formulations for parenteral administration are described in the document, which is a guidebook generally known in all pharmaceutical chemistry fields (Remington's Pharmaceutical Science, 15th Edition, 1975. Mack Publishing Company, Easton, Pennsylvania 18042, Chapter 87: Blaug, Seymour).

The pharmaceutical composition of the present invention is administered in a pharmaceutically effective amount. The term "pharmaceutically effective amount" as used herein refers to an amount sufficient to treat diseases at a reasonable benefit/risk ratio applicable to medical treatment, and an effective dosage level may be determined according to factors including type of diseases of patients, the severity of disease, the activity of drugs, sensitivity to drugs, administration time, administration routes, excretion rate, treatment periods, and simultaneously used drugs, and other factors well known in the medical field. The pharmaceutical composition of the present invention may be administered as an individual therapeutic agent or in combination with other therapeutic agents, may be administered sequentially or simultaneously with therapeutic agents in the related art, and may be administered in a single dose or multiple doses. That is, the total effective amount of the composition of the present invention may be administered to a patient in a single dose or may be administered by a fractionated treatment protocol, in which multiple doses are administered over a long period of time. It is important to administer the composition in a minimum amount that can obtain the maximum effect without any side effects, in consideration of all the aforementioned factors, and this amount may be easily determined by those skilled in the art.

A dosage of the pharmaceutical composition of the present invention varies according to body weight, age, gender, and health status of a patient, age of a patient, diet, administration time, administration method, excretion rate, and the severity of a disease. A daily dosage thereof may be administered parenterally in an amount of preferably 0.01 to 50 mg, and more preferably 0.1 mg to 30 mg per 1 kg of body weight a day based on HSA-Slit3 LRRD2, and a daily dosage thereof may be administered orally in a single dose or multiple doses in an amount of preferably 0.01 to 100 mg, and more preferably 0.01 to 10 mg per 1 kg of body weight a day based on the HSA-Slit3 LRRD2 of the present invention. However, since the effective amount may be increased or decreased depending on the administration route, the severity of obesity, gender, body weight, age, and the like, the dosage is not intended to limit the scope of the present invention in any way.

The pharmaceutical composition of the present invention may be used either alone or in combination with surgery, radiation therapy, hormone therapy, chemotherapy, and methods using a biological response modifier.

The pharmaceutical composition of the present invention may also be provided as a formulation for external application. When the pharmaceutical composition for preventing and treating bone-related diseases according to the present invention is used as a preparation for external application to the skin, the pharmaceutical composition may additionally contain auxiliary agents typically used in the dermatology field, such as any other ingredients typically used in the preparation for external application to the skin, such as a fatty substance, an organic solvent, a solubilizing agent, a thickener and a gelling agent, a softener, an antioxidant, a suspending agent, a stabilizer, a foaming agent, an odorant, a surfactant, water, an ionic emulsifier, a nonionic emulsifier, a filler, a metal ion blocking agent, a chelating agent, a preservative, a vitamin, a blocking agent, a wetting agent, an essential oil, a dye, a pigment, a hydrophilic active agent, a lipophilic active agent, or a lipid vesicle. In addition, the ingredients may be introduced in an amount generally used in the dermatology field.

When the pharmaceutical composition for preventing and treating bone-related diseases according to the present invention is provided as a preparation for external application to the skin, the pharmaceutical composition may be in the form of a formulation such as an ointment, a patch, a gel, a cream, and an aerosol, but is not limited thereto.

The bone-related disease of the present invention refers to a disease that may occur due to an increase in bone resorption or a decrease in bone formation, for example, a decrease in bone mass while bone formation becomes less than bone resorption, and is more preferably any one or more selected from the group consisting of osteoporosis, fractures, bone loss, osteoarthritis, metastatic bone cancer, and Paget's disease, but is not limited thereto.

The present invention also provides a health functional food composition comprising albumin-bound LRRD2 of the Slit3 protein for prevention or alleviation of bone-related diseases. Since the composition of an active ingredient included in the health functional food composition of the present invention and effects thereof are the same as those for the above-described pharmaceutical composition, the description thereof will be omitted.

The health functional food composition according to the present invention can be prepared in various forms by typical methods known in the art. A general food can be prepared by adding the HSA-Slit3 LRRD2 fusion protein of the present invention to, without being limited to, a beverage (including an alcoholic beverage), fruit and a processed food thereof (for example: canned fruit, bottled food, jam, marmalade, and the like), fish, meat and processed food thereof (for example: ham, sausage, corned beef, and the like), bread and noodles (for example: thick wheat noodles, buckwheat noodles, instant noodles, spaghetti, macaroni, and the like), fruit juice, various drinks, cookies, wheat-gluten, dairy products (for example: butter, cheese, and the like), edible vegetable oils, margarine, vegetable protein, retort foods, frozen food and various seasonings (for example: soybean paste, soy sauce, sauce, and the like), and the like. In addition, a nutritional supplement can be prepared by adding the HSA-Slit3 LRRD2 fusion protein of the present invention to, without being limited to, a capsule, a tablet, a pill, and the like. Furthermore, for a health functional food, for example, the HSA-Slit3 LRRD2 fusion protein of the present invention itself is prepared in the form of, without being limited to, tea, juice, and drinks and can be taken by being processed into a liquid, granules, a capsule, and a powder so as to be able to be drunk (health beverage). Further, the HSA-Slit3 LRRD2 fusion protein of the present invention can be used and prepared in the form of a powder or a concentrated liquid so as to be used in the form of a food additive. In addition, the food functional composition of the present invention can be prepared in the form of a composition by mixing the HSA-Slit3 LRRD2 fusion protein of the present invention with an active ingredient known to have effects of preventing bone-related diseases and improving muscular function.

When the HSA-Slit3 LRRD2 fusion protein of the present invention is used as a health beverage, the health beverage composition can contain various flavoring agents or natural carbohydrates, and the like as additional ingredients, such as a typical beverage. The above-described natural carbohydrates may be monosaccharides such as glucose and fructose; disaccharides such as maltose and sucrose; polysaccharides such as dextrin and cyclodextrin; and sugar alcohols such as xylitol, sorbitol, and erythritol. As a sweetener, it is possible to use a natural sweetener such as thaumatin and stevia extract; a synthetic sweetener such as saccharin and aspartame, and the like. The proportion of the natural carbohydrate is generally about 0.01 to 0.04 g, and preferably about 0.02 to 0.03 g per 100 mL of the composition of the present invention.

Furthermore, the HSA-Slit3 LRRD2 fusion protein of the present invention may be contained as an active ingredient of a food composition for prevention or alleviation of bone-related diseases, and the amount thereof is an amount effective to achieve effects of preventing or alleviating bone-related diseases and is not particularly limited, but is preferably 0.01 to 100 wt% based on the total weight of the entire composition. The health functional food composition of the present invention can be prepared by mixing the HSA-Slit3 LRRD2 fusion protein of the present invention with other active ingredients known to have effects on bone-related diseases.

In addition to the aforementioned ingredients, the health functional food composition of the present invention may contain various nutrients, vitamins, electrolytes, flavoring agents, coloring agents, pectic acid, salts of pectic acid, alginic acid, salts of alginic acid, organic acids, protective colloidal thickeners, pH adjusting agents, stabilizers, preservatives, glycerin, alcohols, carbonate agents, and the like. In addition, the health food of the present invention may contain flesh for preparing natural fruit juice, fruit juice beverages, or vegetable beverages. These ingredients may be used either alone or in mixtures thereof. The proportion of these additives is not significantly important, but is generally selected within a range of 0.01 to 0.1 part by weight per 100 parts by weight of the composition of the present invention.

Hereinafter, the present invention will be described in more detail through Examples. These Examples are only for exemplifying the present invention, and it should be obvious to a person with ordinary skill in the art that the scope of the present invention is not to be interpreted as being limited by these Examples.

The abbreviations used in the examples and meanings thereof are as shown in the following Table 1.

[Table 1]

| CL | Systemic plasma clearance |
|---|---|
| $T_{1/2}$ | Terminal half-life |
| $V_{ss}$ | Steady state volume of distribution |
| IV | Intravenous |
| PO | Per oral |
| $C_{max}$ | Maximum plasma concentration observed |
| $T_{max}$ | Time to $C_{max}$ |
| $AUC_{0-\infty}$ | Total area under the plasma concentration time curve from zero to infinity |

(continued)

| AUC$_{0-t}$ | Area under the plasma concentration time curve from zero to the last quantifiable time point |
| --- | --- |
| MRT | Mean residence time |
| BA | Estimated bioavailability |
| BQL | Below Quantification Level |

[Example 1]

**Preparation of HSA-Slit3 LRRD2 fusion protein**

Expression was performed by transforming Expi293F suspension cells with 1.6 mg/ml PC DNA3.1 vector SP cystatin S-HSA-Slit3 LRR D2-FLAG DNA. After cells were cultured to 4.5 to 5 x 10$^6$ cells/ml in 125 ml of a 293F cell suspension and only the medium was replaced with a new medium, transfection was performed by reacting 400 $\mu$l of Expifectamine with 7.5 ml of (A Sample) at room temperature for 5 minutes, reacting 150 ug of DNA with 7.5 ml of Opti-mem (B Sample) at room temperature for 5 minutes, and then mixing A and B Samples to react A and B Samples at room temperature for 20 minutes. After 24 hours, cells were treated by mixing Enhancers 1 and 2, and then cultured for 7 days.

After cells were precipitated from the culture solution cultured for 7 days using a centrifuge at 4°C and 800 rpm for 20 minutes, the supernatant was filtered with a 0.22 $\mu$m filter manufactured by Corning and used. As a resin, an anti-FLAG resin manufactured by Sigma was used. 1.2 ml of the resin was respectively used, and purification was performed at 1 ml/min at 4°C. A washing buffer using Tris glycine (TBS, pH 7.4) was flowed in an amount which is 20-fold higher than that of the resin. For elution, 200 $\mu$l of a FLAG peptide manufactured by Sigma-Aldrich and 9.8 ml of TBS were mixed and used, 8 pieces of 500 $\mu$l per fraction were obtained, protein fractions were collected, concentrated by changing the buffer to DPBS, and then the concentration was measured.

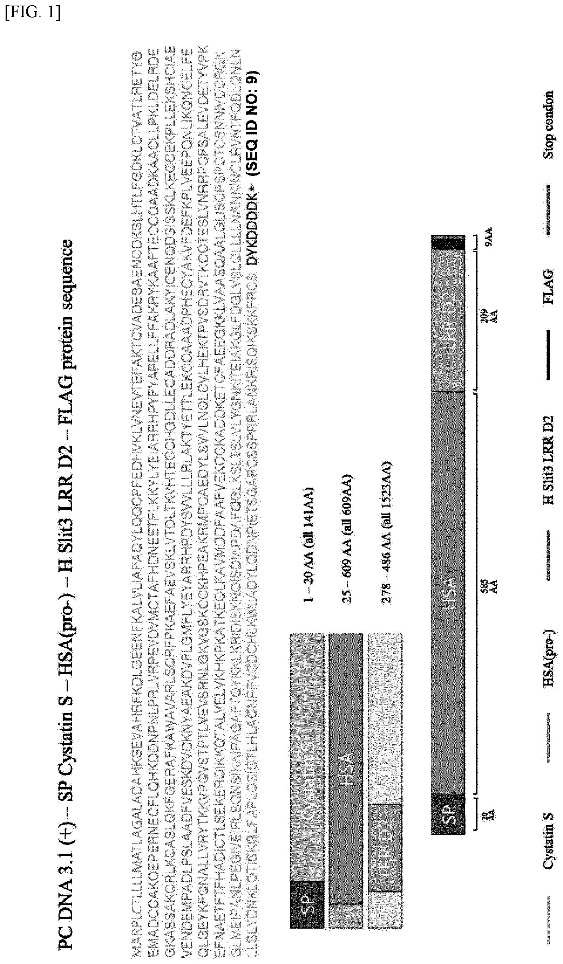

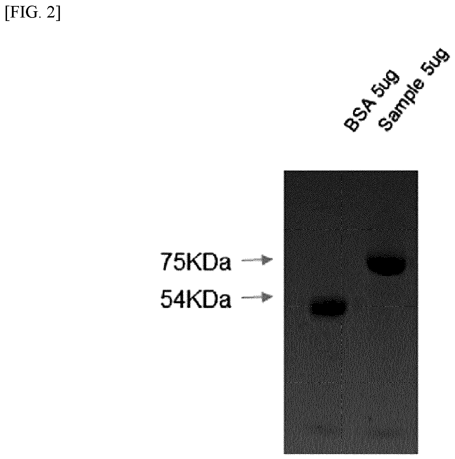

FIG. 2 illustrates the results of performing SDS-PAGE after isolating and purifying the fusion protein by the above process, confirming that the size of the fusion protein illustrated in FIG. 1, which was prepared in the present example, was 75 KDa.

[Example 2]

**Confirmation of receptor binding ability of various forms of HSA-Slit3 LRRD2 fusion proteins**

2-1. Preparation of various forms of HSA-Slit3 LRRD2 fusion proteins

**Based on the preparation method of Example 1, 12 types of various HSA-**Slit3 LRRD2 fusion proteins were prepared as shown in the following Table 2. As a linker, (GGGGS)$_3$ (SEQ ID NO: 6) was used.

[Table 2]

| Type of fusion protein | Terminus bound to HSA | Presenc e or absence of linker | Type of LRRD2 | Final form |
| --- | --- | --- | --- | --- |
| LRRD2-1 | N terminus | None | Fragment (68 a.a.) | HSA-Fragment LRRD2 |
| LRRD2-2 | N terminus | None | Intermediate (130 a.a) | HSA-Intermediate LRRD2 |
| LRRD2-3 | N terminus | None | Full-length (209 a.a) | HSA-Full-length LRRD2 |
| LRRD2-4 | N terminus | Present | Fragment (68 a.a.) | HSA-Linker-Fragment LRRD2 |
| LRRD2-5 | N terminus | Present | Intermediate (130 a.a) | HSA-Linker-Intermediate LRRD2 |
| LRRD2-6 | N terminus | Present | Full-length (209 a.a) | HSA-Linker-Full-length LRRD2 |

(continued)

| Type of fusion protein | Terminus bound to HSA | Presence or absence of linker | Type of LRRD2 | Final form |
|---|---|---|---|---|
| LRRD2-7 | C terminus | None | Fragment (68 a.a.) | Fragment LRRD2-HSA |
| LRRD2-8 | C terminus | None | Intermediate (130 a.a) | Intermediate LRRD2-HSA |
| LRRD2-9 | C terminus | None | Full-length (209 a.a) | Full-length LRRD2-HSA |
| LRRD2-10 | C terminus | Present | Fragment (68 a.a.) | Fragment LRRD2-Linker-HSA |
| LRRD2-11 | C terminus | Present | Intermediate (130 a.a) | Intermediate LRRD2-Linker-HSA |
| LRRD2-12 | C terminus | Present | Full-length (209 a.a) | Full-length LRRD2-Linker-HSA |

The amino acid sequences of the 12 types of HSA-Slit3LRRD2 fusion proteins are shown in Table 3.

[Table 3]

| Type of fusion protein | Amino acid sequence | SEQ ID NO |
|---|---|---|
| LRRD2-1 | MMARPLCTLLLLMATLAGALADAHKSEVA HRFKDLGEENFKALVLIAFAQYLQQCPFEDH VKLVNEVTEFAKTCVADESAENCDKSLHTL FGDKLCTVATLRETYGEMADCCAKQEPERN ECFLQHKDDNPNLPRLVRPEVDVMCTAFHD NEETFLKKYLYEIARRHPYFYAPELLFFAKR YKAAFTECCQAADKAACLLPKLDELRDEGK ASSAKQRLKCASLQKFGERAFKAWAVARLS QRFPKAEFAEVSKLVTDLTKVHTECCHGDL LECADDRADLAKYICENQDSISSKLKECCEK PLLEKSHCIAEVENDEMPADLPSLAADFVES KDVCKNYAEAKDVFLGMFLYEYARRHPDY SVVLLLRLAKTYETTLEKCCAAADPHECYA KVFDEFKPLVEEPQNLIKQNCELFEQLGEYK FQNALLVRYTKKVPQVSTPTLVEVSRNLGK VGSKCCKHPEAKRMPCAEDYLSVVLNQLCV LHEKTPVSDRVTKCCTESLVNRRPCFSALEV DETYVPKEFNAETFTHADICTLSEKERQIKK QTALVELVKHKPKATKEQLKAVMDDFAAF VEKCCKADDKETCFAEEGKKLVAASQAALG LLTSLVLYGNKITEIAKGLFDGLVSLQLLLLN ANKINCLRVNTFQDLQNLNLLSLYDNKLQTI SKGLFADYKDDDDK | 7 |
| LRRD2-2 | MARPLCTLLLLMATLAGALADAHKSEVAHR FKDLGEENFKALVLIAFAQYLQQCPFEDHVK | 8 |

(continued)

| Type of fusion protein | Amino acid sequence | SEQ ID NO |
|---|---|---|
| | LVNEVTEFAKTCVADESAENCDKSLHTLFG DKLCTVATLRETYGEMADCCAKQEPERNEC FLQHKDDNPNLPRLVRPEVDVMCTAFHDNE ETFLKKYLYEIARRHPYFYAPELLFFAKRYK AAFTECCQAADKAACLLPKLDELRDEGKAS SAKQRLKCASLQKFGERAFKAWAVARLSQR FPKAEFAEVSKLVTDLTKVHTECCHGDLLEC ADDRADLAKYICENQDSISSKLKECCEKPLL EKSHCIAEVENDEMPADLPSLAADFVESKDV CKNYAEAKDVFLGMFLYEYARRHPDYSVV LLLRLAKTYETTLEKCCAAADPHECYAKVF DEFKPLVEEPQNLIKQNCELFEQLGEYKFQN ALLVRYTKKVPQVSTPTLVEVSRNLGKVGS KCCKHPEAKRMPCAEDYLSVVLNQLCVLHE KTPVSDRVTKCCTESLVNRRPCFSALEVDET YVPKEFNAETFTHADICTLSEKERQIKKQTA LVELVKHKPKATKEQLKAVMDDFAAFVEK CCKADDKETCFAEEGKKLVAASQAALGLIV EIRLEQNSIKAIPAGAFTQYKKLKRIDISKNQI SDIAPDAFQGLKSLTSLVLYGNKITEIAKGLF DGLVSLQLLLLNANKINCLRVNTFQDLQNL NLLSLYDNKLQTISKGLFAPLQSIQTLHLAQN PDYKDDDDK | |

(continued)

| Type of fusion protein | Amino acid sequence | SEQ ID NO |
|---|---|---|
| LRRD2-3 | MARPLCTLLLLMATLAGALADAHKSEVAHR FKDLGEENFKALVLIAFAQYLQQCPFEDHVK LVNEVTEFAKTCVADESAENCDKSLHTLFG DKLCTVATLRETYGEMADCCAKQEPERNEC FLQHKDDNPNLPRLVRPEVDVMCTAFHDNE ETFLKKYLYEIARRHPYFYAPELLFFAKRYK AAFTECCQAADKAACLLPKLDELRDEGKAS SAKQRLKCASLQKFGERAFKAWAVARLSQR FPKAEFAEVSKLVTDLTKVHTECCHGDLLEC ADDRADLAKYICENQDSISSKLKECCEKPLL EKSHCIAEVENDEMPADLPSLAADFVESKDV CKNYAEAKDVFLGMFLYEYARRHPDYSVV LLLRLAKTYETTLEKCCAAADPHECYAKVF DEFKPLVEEPQNLIKQNCELFEQLGEYKFQN ALLVRYTKKVPQVSTPTLVEVSRNLGKVGS KCCKHPEAKRMPCAEDYLSVVLNQLCVLHE KTPVSDRVTKCCTESLVNRRPCFSALEVDET YVPKEFNAETFTFHADICTLSEKERQIKKQTA LVELVKHKPKATKEQLKAVMDDFAAFVEK CCKADDKETCFAEEGKKLVAASQAALGLIS CPSPCTCSNNIVDCRGKGLMEIPANLPEGIVE IRLEQNSIKAIPAGAFTQYKKLKRIDISKNQIS DIAPDAFQGLKSLTSLVLYGNKITEIAKGLFD GLVSLQLLLLNANKINCLRVNTFQDLQNLNL LSLYDNKLQTISKGLFAPLQSIQTLHLAQNPF VCDCHLKWLADYLQDNPIETSGARCSSPRRL ANKRISQIKSKKFRCSDYKDDDDK | 9 |

(continued)

| Type of fusion protein | Amino acid sequence | SEQ ID NO |
|---|---|---|
| LRRD2-4 | MARPLCTLLLLMATLAGALADAHKSEVAHR FKDLGEENFKALVLIAFAQYLQQCPFEDHVK LVNEVTEFAKTCVADESAENCDKSLHTLFG DKLCTVATLRETYGEMADCCAKQEPERNEC FLQHKDDNPNLPRLVRPEVDVMCTAFHDNE ETFLKKYLYEIARRHPYFYAPELLFFAKRYK AAFTECCQAADKAACLLPKLDELRDEGKAS SAKQRLKCASLQKFGERAFKAWAVARLSQR FPKAEFAEVSKLVTDLTKVHTECCHGDLLEC ADDRADLAKYICENQDSISSKLKECCEKPLL EKSHCIAEVENDEMPADLPSLAADFVESKDV CKNYAEAKDVFLGMFLYEYARRHPDYSVV LLLRLAKTYETTLEKCCAAADPHECYAKVF DEFKPLVEEPQNLIKQNCELFEQLGEYKFQN ALLVRYTKKVPQVSTPTLVEVSRNLGKVGS KCCKHPEAKRMPCAEDYLSVVLNQLCVLHE KTPVSDRVTKCCTESLVNRRPCFSALEVDET YVPKEFNAETFTFHADICTLSEKERQIKKQTA LVELVKHKPKATKEQLKAVMDDFAAFVEK CCKADDKETCFAEEGKKLVAASQAALGL<u>GG GGSGGGGSGGGGS</u>LTSLVLYGNKITEIAKGL FDGLVSLQLLLLNANKINCLRVNTFQDLQNL NLLSLYDNKLQTISKGLFADYKDDDDK | 10 |

(continued)

| Type of fusion protein | Amino acid sequence | SEQ ID NO |
|---|---|---|
| LRRD2-5 | MARPLCTLLLLMATLAGALADAHKSEVAHR FKDLGEENFKALVLIAFAQYLQQCPFEDHVK LVNEVTEFAKTCVADESAENCDKSLHTLFG DKLCTVATLRETYGEMADCCAKQEPERNEC FLQHKDDNPNLPRLVRPEVDVMCTAFHDNE ETFLKKYLYEIARRHPYFYAPELLFFAKRYK AAFTECCQAADKAACLLPKLDELRDEGKAS SAKQRLKCASLQKFGERAFKAWAVARLSQR FPKAEFAEVSKLVTDLTKVHTECCHGDLLEC ADDRADLAKYICENQDSISSKLKECCEKPLL EKSHCIAEVENDEMPADLPSLAADFVESKDV CKNYAEAKDVFLGMFLYEYARRHPDYSVV LLLRLAKTYETTLEKCCAAADPHECYAKVF DEFKPLVEEPQNLIKQNCELFEQLGEYKFQN ALLVRYTKKVPQVSTPTLVEVSRNLGKVGS KCCKHPEAKRMPCAEDYLSVVLNQLCVLHE KTPVSDRVTKCCTESLVNRRPCFSALEVDET YVPKEFNAETFTFHADICTLSEKERQIKKQTA LVELVKHKPKATKEQLKAVMDDFAAFVEK CCKADDKETCFAEEGKKLVAASQAALGL<u>GG GGSGGGGSGGGGS</u>IVEIRLEQNSIKAIPAGAF TQYKKLKRIDISKNQISDIAPDAFQGLKSLTS LVLYGNKITEIAKGLFDGLVSLQLLLLNANKI NCLRVNTFQDLQNLNLLSLYDNKLQTISKGL FAPLQSIQTLHLAQNPDYKDDDDK | 11 |

(continued)

| Type of fusion protein | Amino acid sequence | SEQ ID NO |
|---|---|---|
| LRRD2-6 | MARPLCTLLLLMATLAGALADAHKSEVAHR FKDLGEENFKALVLIAFAQYLQQCPFEDHVK LVNEVTEFAKTCVADESAENCDKSLHTLFG DKLCTVATLRETYGEMADCCAKQEPERNEC FLQHKDDNPNLPRLVRPEVDVMCTAFHDNE ETFLKKYLYEIARRHPYFYAPELLFFAKRYK AAFTECCQAADKAACLLPKLDELRDEGKAS SAKQRLKCASLQKFGERAFKAWAVARLSQR FPKAEFAEVSKLVTDLTKVHTECCHGDLLEC ADDRADLAKYICENQDSISSKLKECCEKPLL EKSHCIAEVENDEMPADLPSLAADFVESKDV CKNYAEAKDVFLGMFLYEYARRHPDYSVV LLLRLAKTYETTLEKCCAAADPHECYAKVF DEFKPLVEEPQNLIKQNCELFEQLGEYKFQN ALLVRYTKKVPQVSTPTLVEVSRNLGKVGS KCCKHPEAKRMPCAEDYLSVVLNQLCVLHE KTPVSDRVTKCCTESLVNRRPCFSALEVDET YVPKEFNAETFTFHADICTLSEKERQIKKQTA LVELVKHKPKATKEQLKAVMDDFAAFVEK CCKADDKETCFAEEGKKLVAASQAALGL<u>GG GGSGGGGSGGGGS</u>ISCPSPCTCSNNIVDCRG KGLMEIPANLPEGIVEIRLEQNSIKAIPAGAFT QYKKLKRIDISKNQISDIAPDAFQGLKSLTSL VLYGNKITEIAKGLFDGLVSLQLLLLNANKI NCLRVNTFQDLQNLNLLSLYDNKLQTISKGL FAPLQSIQTLHLAQNPFVCDCHLKWLADYL QDNPIETSGARCSSPRRLANKRISQIKSKKFR CSDYKDDDDK | 12 |

(continued)

| Type of fusion protein | Amino acid sequence | SEQ ID NO |
|---|---|---|
| LRRD2-7 | MARPLCTLLLLMATLAGALALTSLVLYGNK ITEIAKGLFDGLVSLQLLLLNANKINCLRVNT FQDLQNLNLLSLYDNKLQTISKGLFADAHKS EVAHRFKDLGEENFKALVLIAFAQYLQQCPF EDHVKLVNEVTEFAKTCVADESAENCDKSL HTLFGDKLCTVATLRETYGEMADCCAKQEP ERNECFLQHKDDNPNLPRLVRPEVDVMCTA FHDNEETFLKKYLYEIARRHPYFYAPELLFF AKRYKAAFTECCQAADKAACLLPKLDELRD EGKASSAKQRLKCASLQKFGERAFKAWAV ARLSQRFPKAEFAEVSKLVTDLTKVHTECCH GDLLECADDRADLAKYICENQDSISSKLKEC CEKPLLEKSHCIAEVENDEMPADLPSLAADF VESKDVCKNYAEAKDVFLGMFLYEYARRH PDYSVVLLLRLAKTYETTLEKCCAAADPHE CYAKVFDEFKPLVEEPQNLIKQNCELFEQLG EYKFQNALLVRYTKKVPQVSTPTLVEVSRN LGKVGSKCCKHPEAKRMPCAEDYLSVVLNQ LCVLHEKTPVSDRVTKCCTESLVNRRPCFSA LEVDETYVPKEFNAETFTFHADICTLSEKER QIKKQTALVELVKHKPKATKEQLKAVMDDF<br><br>AAFVEKCCKADDKETCFAEEGKKLVAASQA ALGL**GGGGSGGGGSGGGGS**DYKDDDDK | 13 |

(continued)

| Type of fusion protein | Amino acid sequence | SEQ ID NO |
|---|---|---|
| LRRD2-8 | MARPLCTLLLLMATLAGALAIVEIRLEQNSI KAIPAGAFTQYKKLKRIDISKNQISDIAPDAF QGLKSLTSLVLYGNKITEIAKGLFDGLVSLQ LLLLNANKINCLRVNTFQDLQNLNLLSLYDN KLQTISKGLFAPLQSIQTLHLAQNPDAHKSE VAHRFKDLGEENFKALVLIAFAQYLQQCPFE DHVKLVNEVTEFAKTCVADESAENCDKSLH TLFGDKLCTVATLRETYGEMADCCAKQEPE RNECFLQHKDDNPNLPRLVRPEVDVMCTAF HDNEETFLKKYLYEIARRHPYFYAPELLFFA KRYKAAFTECCQAADKAACLLPKLDELRDE GKASSAKQRLKCASLQKFGERAFKAWAVA RLSQRFPKAEFAEVSKLVTDLTKVHTECCHG DLLECADDRADLAKYICENQDSISSKLKECC EKPLLEKSHCIAEVENDEMPADLPSLAADFV ESKDVCKNYAEAKDVFLGMFLYEYARRHP DYSVVLLLRLAKTYETTLEKCCAAADPHEC YAKVFDEFKPLVEEPQNLIKQNCELFEQLGE YKFQNALLVRYTKKVPQVSTPTLVEVSRNL GKVGSKCCKHPEAKRMPCAEDYLSVVLNQL CVLHEKTPVSDRVTKCCTESLVNRRPCFSAL EVDETYVPKEFNAETFTFHADICTLSEKERQI KKQTALVELVKHKPKATKEQLKAVMDDFA AFVEKCCKADDKETCFAEEGKKLVAASQAA LGL**GGGGSGGGGSGGGG**SDYKDDDDK | 14 |

(continued)

| Type of fusion protein | Amino acid sequence | SEQ ID NO |
|---|---|---|
| LRRD2-9 | MARPLCTLLLLMATLAGALAISCPSPCTCSN NIVDCRGKGLMEIPANLPEGIVEIRLEQNSIK AIPAGAFTQYKKLKRIDISKNQISDIAPDAFQ GLKSLTSLVLYGNKITEIAKGLFDGLVSLQLL LLNANKINCLRVNTFQDLQNLNLLSLYDNK LQTISKGLFAPLQSIQTLHLAQNPFVCDCHLK WLADYLQDNPIETSGARCSSPRRLANKRISQI KSKKFRCSDAHKSEVAHRFKDLGEENFKAL VLIAFAQYLQQCPFEDHVKLVNEVTEFAKTC VADESAENCDKSLHTLFGDKLCTVATLRET YGEMADCCAKQEPERNECFLQHKDDNPNLP RLVRPEVDVMCTAFHDNEETFLKKYLYEIA RRHPYFYAPELLFFAKRYKAAFTECCQAAD KAACLLPKLDELRDEGKASSAKQRLKCASL QKFGERAFKAWAVARLSQRFPKAEFAEVSK LVTDLTKVHTECCHGDLLECADDRADLAKY ICENQDSISSKLKECCEKPLLEKSHCIAEVEN DEMPADLPSLAADFVESKDVCKNYAEAKDV FLGMFLYEYARRHPDYSVVLLLRLAKTYET TLEKCCAAADPHECYAKVFDEFKPLVEEPQ NLIKQNCELFEQLGEYKFQNALLVRYTKKVP QVSTPTLVEVSRNLGKVGSKCCKHPEAKRM<br><br>PCAEDYLSVVLNQLCVLHEKTPVSDRVTKC CTESLVNRRPCFSALEVDETYVPKEFNAETF TFHADICTLSEKERQIKKQTALVELVKHKPK ATKEQLKAVMDDFAAFVEKCCKADDKETC FAEEGKKLVAASQAALGL**GGGGSGGGGSG GGGS**DYKDDDDK | 15 |

(continued)

| Type of fusion protein | Amino acid sequence | SEQ ID NO |
|---|---|---|
| LRRD2-10 | MARPLCTLLLLMATLAGALALTSLVLYGNK ITEIAKGLFDGLVSLQLLLLNANKINCLRVNT FQDLQNLNLLSLYDNKLQTISKGLFAGGGGS GGGGSGGGGSDAHKSEVAHRFKDLGEENFK ALVLIAFAQYLQQCPFEDHVKLVNEVTEFAK TCVADESAENCDKSLHTLFGDKLCTVATLR ETYGEMADCCAKQEPERNECFLQHKDDNPN LPRLVRPEVDVMCTAFHDNEETFLKKYLYEI ARRHPYFYAPELLFFAKRYKAAFTECCQAA DKAACLLPKLDELRDEGKASSAKQRLKCAS LQKFGERAFKAWAVARLSQRFPKAEFAEVS KLVTDLTKVHTECCHGDLLECADDRADLAK YICENQDSISSKLKECCEKPLLEKSHCIAEVE NDEMPADLPSLAADFVESKDVCKNYAEAKD VFLGMFLYEYARRHPDYSVVLLLRLAKTYE TTLEKCCAAADPHECYAKVFDEFKPLVEEPQ NLIKQNCELFEQLGEYKFQNALLVRYTKKVP QVSTPTLVEVSRNLGKVGSKCCKHPEAKRM PCAEDYLSVVLNQLCVLHEKTPVSDRVTKC CTESLVNRRPCFSALEVDETYVPKEFNAETF TFHADICTLSEKERQIKKQTALVELVKHPK ATKEQLKAVMDDFAAFVEKCCKADDKETC FAEEGKKLVAASQAALGL**GGGGSGGGGSG GGGS**DYKDDDDK | 16 |
| LRRD2-11 | MARPLCTLLLLMATLAGALAIVEIRLEQNSI KAIPAGAFTQYKKLKRIDISKNQISDIAPDAF QGLKSLTSLVLYGNKITEIAKGLFDGLVSLQ LLLLNANKINCLRVNTFQDLQNLNLLSLYDN KLQTISKGLFAPLQSIQTLHLAQNPGGGGSG GGGSGGGGSDAHKSEVAHRFKDLGEENFKA LVLIAFAQYLQQCPFEDHVKLVNEVTEFAKT CVADESAENCDKSLHTLFGDKLCTVATLRE TYGEMADCCAKQEPERNECFLQHKDDNPNL PRLVRPEVDVMCTAFHDNEETFLKKYLYEIA RRHPYFYAPELLFFAKRYKAAFTECCQAAD KAACLLPKLDELRDEGKASSAKQRLKCASL QKFGERAFKAWAVARLSQRFPKAEFAEVSK LVTDLTKVHTECCHGDLLECADDRADLAKY ICENQDSISSKLKECCEKPLLEKSHCIAEVEN DEMPADLPSLAADFVESKDVCKNYAEAKDV FLGMFLYEYARRHPDYSVVLLLRLAKTYET TLEKCCAAADPHECYAKVFDEFKPLVEEPQ NLIKQNCELFEQLGEYKFQNALLVRYTKKVP | 17 |

(continued)

| Type of fusion protein | Amino acid sequence | SEQ ID NO |
|---|---|---|
| | QVSTPTLVEVSRNLGKVGSKCCKHPEAKRM PCAEDYLSVVLNQLCVLHEKTPVSDRVTKC CTESLVNRRPCFSALEVDETYVPKEFNAETF TFHADICTLSEKERQIKKQTALVELVKHKPK ATKEQLKAVMDDFAAFVEKCCKADDKETC FAEEGKKLVAASQAALGL**GGGGSGGGGSG GGGS**DYKDDDDK | |
| LRRD2-12 | MARPLCTLLLLMATLAGALAISCPSPCTCSN NIVDCRGKGLMEIPANLPEGIVEIRLEQNSIK AIPAGAFTQYKKLKRIDISKNQISDIAPDAFQ GLKSLTSLVLYGNKITEIAKGLFDGLVSLQLL LLNANKINCLRVNTFQDLQNLNLLSLYDNK LQTISKGLFAPLQSIQTLHLAQNPFVCDCHLK WLADYLQDNPIETSGARCSSPRRLANKRISQI KSKKFRCS<u>GGGGSGGGGSGGGGS</u>DAHKSEV AHRFKDLGEENFKALVLIAFAQYLQQCPFED HVKLVNEVTEFAKTCVADESAENCDKSLHT LFGDKLCTVATLRETYGEMADCCAKQEPER NECFLQHKDDNPNLPRLVRPEVDVMCTAFH DNEETFLKKYLYEIARRHPYFYAPELLFFAK RYKAAFTECCQAADKAACLLPKLDELRDEG KASSAKQRLKCASLQKFGERAFKAWAVARL SQRFPKAEFAEVSKLVTDLTKVHTECCHGDL LECADDRADLAKYICENQDSISSKLKECCEK PLLEKSHCIAEVENDEMPADLPSLAADFVES KDVCKNYAEAKDVFLGMFLYEYARRHPDY SVVLLLRLAKTYETTLEKCCAAADPHECYA KVFDEFKPLVEEPQNLIKQNCELFEQLGEYK FQNALLVRYTKKVPQVSTPTLVEVSRNLGK VGSKCCKHPEAKRMPCAEDYLSVVLNQLCV LHEKTPVSDRVTKCCTESLVNRRPCFSALEV DETYVPKEFNAETFTFHADICTLSEKERQIKK QTALVELVKHKPKATKEQLKAVMDDFAAF VEKCCKADDKETCFAEEGKKLVAASQAALG L**GGGGSGGGGSGGGGS**DYKDDDDK | 18 |

The underlined sequence in Table 3 is a GS linker linking HSA and LRRD2, and the bold sequence is a GS linker linking a sequence added to the C-terminus in order to express the fusion protein in its final form.

2-2. Confirmation of receptor binding ability of various forms of HSA-Slit3 LRRD2 fusion proteins

The action of Slit3 LRRD2 on bone cells is mediated through Robol and Robo2 receptors. Therefore, in the present example, the Robol receptor binding ability of the 12 types of HSA-Slit3 LRRD2 fusion proteins prepared in Example 2-1 was confirmed. The binding ability of the 12 types of HSA-Slit3 LRRD2 fusion proteins to the receptor was quantified using an ELISA system. Detailed conditions are as follows.

96-well Maxisorp microtiter plates (manufactured by NUNC) were coated with the 12 types of HSA-Slit3 LRRD2 fusion proteins at 4°C for 18 hours at 0, 1, 10, 100, and 1000 nM per well, in consideration of the molecular weight. The coated material was washed three times using PBS containing 0.05% Tween 20 (PBST). Blocking was performed with PBST supplemented with 1% BSA at room temperature for 2 hours to block non-specific binding. The coated material was washed three times with PBST to remove a blocking buffer. After washing, 30 ug of a protein obtained from an osteoblastic cell line, MC3T3-E1, was allowed to adhere (lysis buffer: 0.5% NP40, 50 mM Tris pH 7.5, 150 mM NaCl, ImM EDTA, 0.2 mM NaF, 1 mM $Na_3VO_4$, 1 mM DTT, 1 mM PMSF, and a proteinase inhibitor cocktail) at room temperature for 2 hours. After washing three times with PBST, a Robol antibody (abcam: ab7279) diluted with 0.1% BSA at 1:1000 was adhered thereto at room temperature for 2 hours. After washing three times with PBST, an HRP-binding antibody (cell signaling: 7074) diluted with 0.1% BSA at 1:2000 was adhered thereto at room temperature for 2 hours. After washing five times with PBST, a reaction was performed with a TMB solution at 37°C for 30 minutes. To stop the reaction, 100 $\mu$l of 1 N $H_2SO_4$ was used, and absorbance was measured at 450 nm.

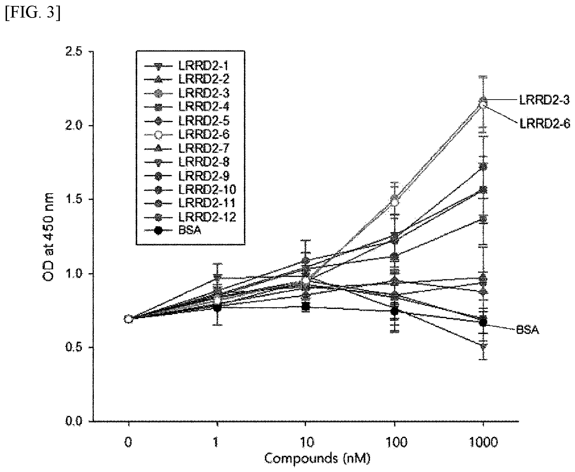

As a result, as illustrated in FIG. 3, it was confirmed that the receptor binding ability of LRRD2-3 and LRRD2-6 was the best.

[Example 3]

## Confirmation of cytological efficacy of various forms of HSA-Slit3 LRRD2 fusion proteins

In the present example, the cytological efficacy of the 12 types of HSA-Slit3LRRD2 fusion proteins prepared in Example 2-1 was confirmed by observing the osteoblast migration ability and the osteoclast differentiation ability according to the treatment of the 12 types of HSA-Slit3 LRRD2 fusion proteins.

### 3-1. Measurement of osteoblast migration ability

A Boyden chamber system (Transwell, 8 um pores) was used to measure the cell migration ability. After an osteoblastic cell line MC3T3-E1 ($1\times10^5$) was diluted in an MEM alpha medium supplemented with 0.2% FBS and attached to an inner chamber for 6 hours, an outer chamber was treated with a drug for 24 hours. After invaded cells were treated with a crystal violet solution containing a fixing solution for 10 minutes, the number of cells was measured under an optical microscope.

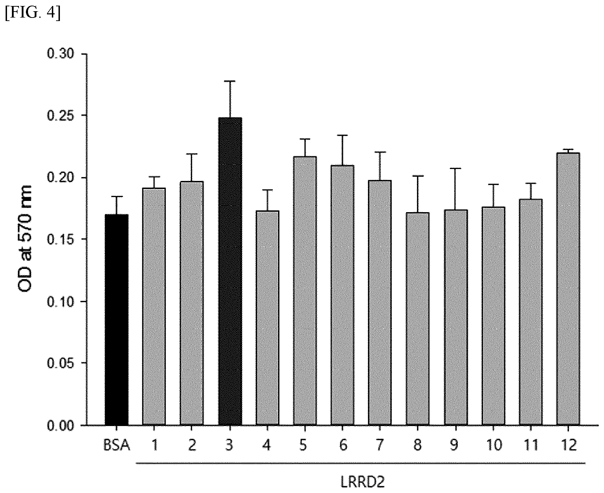

As a result, as illustrated in FIG. 4, it was confirmed that the osteoblast migration ability of LRRD2-3 was the best.

### 3-2. Measurement of differentiation ability of osteoclasts

Osteoclast progenitor cells were extracted from the femurs and tibias of 6-week-old ICR mice, and then cultured in an incubator at 37°C for 18 hours. Only floating cells were collected and treated with 30 ng/ml M-CSF and 30 ng/ml RANKL to differentiate into osteoclasts. After 4 days, the cells were reacted with a TRAP staining solution (leukocyte acid phosphatase) for 10 minutes, and then the number of cells was measured by considering multinucleated cells having 3 or more nuclei stained with TRAP as osteoclasts under an optical microscope.

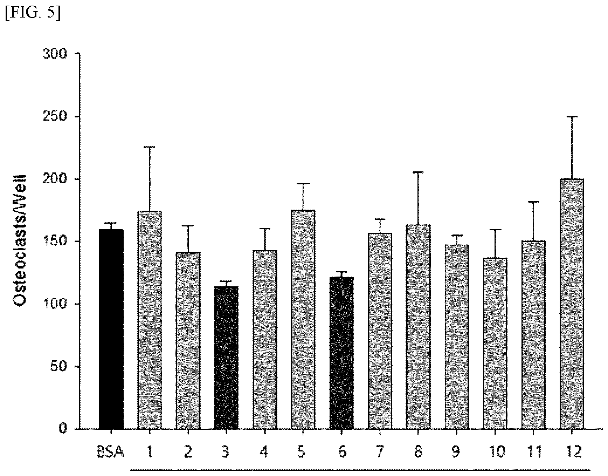

As a result, as illustrated in FIG. 5, it was confirmed that the osteoclast differentiation inhibiting ability of LRRD2-3 and LRRD2-6 was better than the remaining 10 types of HSA-Slit3 LRRD2 fusion proteins.

[Example 4]

## Confirmation of cytological efficacy of HSA-Slit3 LRRD2 fusion protein

In the present example, based on the results of Examples 2 and 3, LRRD2-3, which has the best efficacy in cells, was selected, and observed by comparing its osteoblast migration ability, b-catenin activity of osteoblasts, and osteoclast differentiation inhibiting ability with those of albumin-unbound Slit3 LRRD2.

### 4-1. Measurement of osteoblast migration ability

A Boyden chamber system (Transwell, 8 um pores) was used to measure the migration ability of cells. After an osteoblastic cell line MC3T3-E1 ($1\times10^5$) was diluted in an MEM alpha medium supplemented with 0.2% FBS and attached to an inner chamber for 6 hours, an outer chamber was treated with a drug for 24 hours. After invaded cells were treated with a crystal violet solution containing a fixing solution for 10 minutes, the number of cells was measured under an optical microscope.

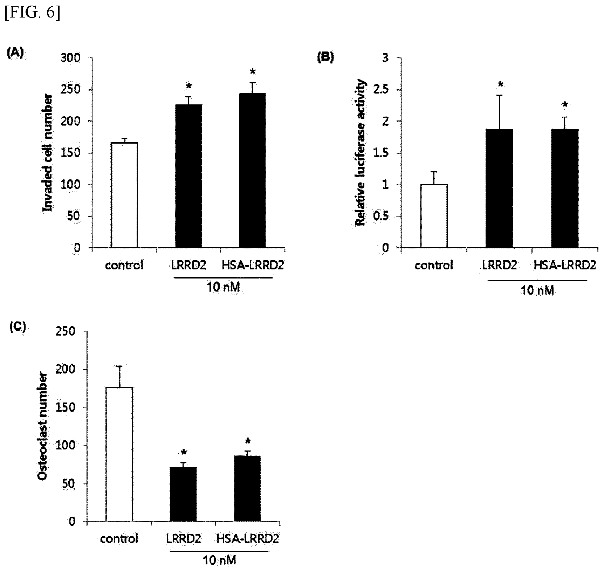

As a result, as illustrated in FIG. 6A, LRRD2-3 promoted osteoblast migration ability to the same extent as albumin-unbound Slit3 LRRD2.

4-2. Measurement of b-catenin activity of osteoblasts

After an MC3T3-E1 cell line ($2 \times 10^4$ cells/well) was cultured in a 24-well plate for 18 hours, 100 ng of 8x Super-TOPFlash and 10 ng of a Renilla reporter plasmid were transfected into the cells. After 48 hours, luciferase activity was measured using a dual luciferase reporter assay kit.

As a result, as illustrated in FIG. 6B, LRRD2-3 activated b-catenin of osteoblasts to the same extent as albumin-unbound Slit3 LRRD2.

4-3. Measurement of differentiation ability of osteoclasts

Osteoclast progenitor cells were extracted from the femurs and tibias of 6-week-old ICR mice, and then cultured in an incubator at 37°C for 18 hours. Only floating cells were collected and treated with 30 ng/ml M-CSF and 30 ng/ml RANKL to differentiate into osteoclasts. After 4 days, the cells were reacted with a TRAP staining solution (leukocyte acid phosphatase) for 10 minutes, and then the number of cells was measured by considering multinucleated cells having 3 or more nuclei stained with TRAP as osteoclasts under an optical microscope.

As a result, as illustrated in FIG. 6C, LRRD2-3 suppressed osteoclast differentiation to the same extent as albumin-unbound Slit3 LRRD2.

[Example 5]

**Pharmacokinetic studies of Slit3 LRRD2 and HSA-Slit3 LRRD2 fusion proteins in mice**

A pharmacokinetic study is a part of new drug development processes, and aims to obtain information on the absorption, distribution, metabolism and excretion of a test drug by assessing changes in drug concentration in the body over time. In the present example, pharmacokinetic properties were confirmed in mice after a single intravenous administration of Slit3 LRRD2-3 and HSA-Slit3 LRRD2 fusion protein (LRRD2-3).

5-1. Chemicals and solvents

The carbamazepine used in this example was purchased from Sigma Aldrich, and HPLC grade acetonitrile and methanol were purchased from J.T. Baker.

5-2. Animals and administration conditions

In the present example, ICR-based male mice (6 weeks old, Orient Bio Co., Ltd., Seongnam, Republic of Korea) with a body weight ranging from 30 to 32.5 g were used. Mice were fasted for 4 hours before the experiment and fasting was maintained for up to 4 hours after administration. The breeding place was given12 hours each of light and dark, and an appropriate temperature (20 to 25°C) and humidity (40 to 60%) were maintained.

[Table 4]

| Pharmacokinetic test | | |
|---|---|---|
| Administered material | Number of animals | Administration dose |
| Slit3 LRRD2 | 4 | 10 mg/kg |
| HSA-Slit3 LRRD2 (LRRD2-3) | 3 | 35 mg/kg |
| Total | 7 | - |

Slit3 LRRD2 was prepared by being dissolved in PBS at a dose of 1 mg/mL. HSA-Slit3 LRRD2 (LRRD2-3) was prepared by being dissolved in PBS at a dose of 3.5 mg/mL (1 mg/mL for Slit3 LRRD2) in consideration of the molecular weight. The dose was 10 mL/kg in both groups, and the prepared solution was administered through the left caudal vein.

5-3. Pharmacokinetic test

In the case of the pharmacokinetic test, fasted mice were administered Slit3 LRRD2 and HSA-Slit3 LRRD2 (LRRD2-3) at a dose of 10 mg/kg and 35 mg/kg, respectively, through the caudal vein. After administration, mice were fixed by hand at 0.05, 0.12, 0.33, 1, 3, 7, 10, 24, 48, and 72 hours, respectively, and then 70 μL of blood was collected from the right orbital venous plexus using heparin-coated capillary tubes. The collected blood was centrifuged for 5 minutes and then stored frozen at -20°C until plasma was isolated and analyzed.

5-4. Analysis method

The concentration of Slit3 LRRD2 in plasma samples was quantified using an HPLC/MS/MS system. Before sample pretreatment, plasma samples were purified using Ni-NTA magnetic beads. After purified Slit3 LRRD2 and HSA-Slit3 LRRD2 (LRRD2-3) were denatured by adding 6M urea and 18 mM dithiothreitol (DTT) thereto, alkylation was induced using 225 mM iodine acetamide. Then, to obtain a signature peptide, 850 ng of recombinant porcine trypsin (V5117, Promega, Madison, WI, USA) was added thereto, and the resulting mixture was reacted in a water bath set at 37°C for 24 hours. After 50 μL of 3% formic acid dissolved in MeOH was added to 70 μL of a trypsin digestion product produced after the reaction, the mixed sample was suspended using a vortex mixer for 10 minutes, centrifuged at 13,500 rpm for 10 minutes, and 160 μL of the supernatant was taken and transferred to an analysis vessel, and 5 μL of the transferred supernatant was injected into an HPLC MSMS system to perform analysis.

Detailed analysis conditions are as follows.

- HPLC system: Agilent 1100 (Agilent Technologies, Santa Clara, CA)
- Column: ZORBAX® C$_8$ 3.5 μm, 2.1*50 mm (Agilent)
- Mobile phase:

  A: 0.1% formic acid dissolved in distilled water
  B: Acetonitrile

(Isocratic elution)

| Time | $0 \rightarrow 0.1 \rightarrow 1.0 \rightarrow 1.5 \rightarrow 2.5 \rightarrow 3 \rightarrow 5$ |
|---|---|
| B (%) | $5 \rightarrow 5 \rightarrow 5 \rightarrow 95 \rightarrow 95 \rightarrow 5 \rightarrow 5$ |

- Flow rate: 300 μL/min
- Temperature: 20°C in column, and 10°C in autosampler tray
- Runtime: 5 minutes
- Detection: Tandem quadrupole mass spectrometer (API 4000, QTRAP®, Applied Biosystems/MDS SCIEX, Foster City, CA, USA)
- Curtain gas: 20 psi
- Ion source gas 1: 50 psi
- Ion source gas 2: 60 psi
- Ionspray voltage: 5500 V
- Temperature: 600°C
- Multiple-reaction-monitoring (MRM) mode: Positive

The molecular ions of a Silt3 LRRD2 signature peptide (P6) were fragmented by a collision energy of 23 V, and a collision gas was set to 'medium (8 psi)' in the equipment. Ions were detected in the ESI-positive MRM mode, and P6 was quantified from 587.97 to 491.50 in units of m/z. Detected peaks were integrated using Analyst software version 1.4.2 (Applied Biosystems/MDS SCIEX). A quantifiable range of Silt3 LRRD2 in plasma was 1 to 100 μg/mL, and that of HSA-Silt3 LRRD2 (LRRD2-3) was 3 to 100 μg/mL. In the corresponding analysis, Slit3 LRRD2 showed a peak retention time of 3.29 minutes.

5-5. Data analysis

The concentration of CNC00000 in plasma over time was determined using the LC-MS/MS analysis method described in Example 5-4, and pharmacokinetic parameters (PK parameters) were calculated using noncompartmental analysis of WinNonlin® 4.2 (Pharsight Corp., Cary, NC, USA) software. The maximum concentration ($C_{max}$) and the maximum concentration arrival time ($T_{max}$) were temporally calculated from a curve according to the blood drug concentration vs. time, and the elimination rate constant ($K_e$) was calculated by a linear regression analysis in the terminal phase of the log scale. The half-life ($T_{1/2}$) was calculated by dividing LN2 by $K_e$, and an area under the curve of blood drug concentration vs. time ($AUC_{0-\infty}$) and an area under the curve of blood drug moment vs. time ($AUMC_{0-\infty}$) were calculated by the linear trapezoidal rule and the standard area extrapolation method. Clearance (CL) and steady state volume of distribution (Vss) were calculated by the following [Equation 1] to [Equation 3]:

[Equation 1]

$$CL = \frac{Dose}{AUC_{0-\infty}}$$

[Equation 2]

$$V_{ss} = MRT \times CL$$

[Equation 3]

$$MRT = \frac{AUMC_{0-\infty}}{AUC_{0-\infty}}$$

5-6. Results

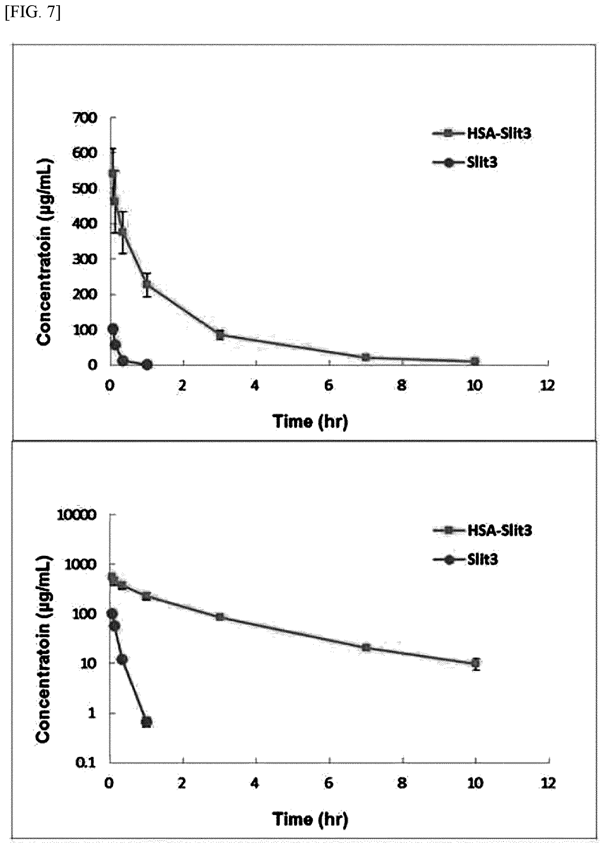

The concentrations of Slit3 LRRD2 and HSA-Slit3 LRRD2 (LRRD2-3) in plasma over time are shown in FIG. 7 and Tables 5 and 6, and pharmacokinetic parameters are shown in Table 6. The related parameters and all values were calculated for each individual and then averaged. Referring to the blood concentration pattern and animal experiment record over time, any abnormal populations were excluded from the data analysis, and the experimental group used for data analysis was set to have at least n = 3 or more.

[Table 5]

| Plasma concentration after intravenous administration of Slit3 | | | | | |
|---|---|---|---|---|---|
| Plasma concentration of Slit3 ($\mu$g/mL) | | | | | |
| Time (h) | #1 | #2 | #3 | #4 | mean | S.D. |
| 0.05 | 122 | 90.9 | 97.6 | 96.7 | 102 | 13.8 |
| 0.12 | 62.6 | 61.4 | 47.9 | 59.7 | 57.9 | 6.77 |
| 0.33 | 14.1 | 11.4 | 12.1 | 11.0 | 12.2 | 1.38 |
| 1 | 0.66 | 0.48 | 0.84 | 0.71 | 0.67 | 0.15 |
| 3 | BQL | BQL | BQL | BQL | 0.000 | - |
| 7 | BQL | BQL | BQL | BQL | 0.000 | - |
| 10 | BQL | BQL | BQL | BQL | 0.000 | - |
| 24 | BQL | BQL | BQL | BQL | 0.000 | - |
| *BQL: When it is less than the quantification limit, it is treated as "0". | | | | | |

[Table 6]

| Plasma concentration after intravenous administration of HSA-Slit3 (LRRD2-3) | | | | | |
|---|---|---|---|---|---|
| Plasma conce ntration of Slit 3 ($\mu$g/mL) | | | | | |
| Time (h) | #6 | #7 | #8 | mean | S.D. |
| 0.05 | 587 | 460 | 577 | 541 | 70.6 |
| 0.12 | 539 | 366 | 480 | 462 | 87.9 |
| 0.33 | 355 | 327 | 441 | 374 | 59.4 |
| 1 | 217 | 199 | 262 | 226 | 32.4 |
| 3 | 82.8 | 73.6 | 98.9 | 85.1 | 12.8 |
| 7 | 18.9 | 18.5 | 23.4 | 20.3 | 2.72 |
| 10 | 9.71 | 7.64 | 12.5 | 9.95 | 2.44 |
| 24 | BQL | BQL | BQL | 0.000 | - |
| *BQL: When it is less than the quantification limit, it is treated as "0". | | | | | |

As confirmed in the following Table 7, the HSA-Slit3 LRRD2 fusion protein (LRRD2-3) showed an approximately 14-fold improved half-life compared to Slit3 LRRD2.

[Table 7]

| Parameter | Slit3 LRRD2 | HSA-Slit3 LRRD2 (LRRD2-3) |
|---|---|---|
| | Average S.D. | Average S.D. |
| $T_{max}$(h) | $0.050 \pm 0.000$ | $0.050 \pm 0.000$ |
| $C_0$ ($\mu$g/mL) | 153.9 33.25 | 607.8 59.87 |
| $C_{max}$ ($\mu$g/mL) | $101.8 \pm 13.79$ | $541.3 \pm 70.61$ |
| $T_{1/2}$ (h) | $0.139 \pm 0.012$ | $1.993 \pm 0.147$ |
| $AUC_{all}$ ($\mu$g·h/mL) | $23.63 \pm 2.534$ | $919.9 \pm 131.2$ |
| $AUC_{inf}$ ($\mu$g·h/mL) | $23.77 \pm 2.530$ | $948.1 \pm 136.1$ |
| CL (mL/h/kg) | $424.0 \pm 40.88$ | $10.69 \pm 1.504$ |
| $V_{ss}$ (mL/kg) | $61.46 \pm 7.368$ | $24.1 \pm 3.293$ |

[Example 6]

**Confirmation of *in vivo* efficacy of HSA-Slit3 LRRD2 fusion protein**

12-week-old SCID mice were treated with albumin-unbound Slit3 LRRD2 or the HSA-Slit3 LRRD2 fusion protein (LRRD2-3) for 4 weeks. Each drug was administered by intravenous injection once daily, five times per week, and Slit3 LRRD2 and the HSA-Slit3 LRRD2 fusion protein (LRRD2-3) were injected daily in a dose of 10 mg and 37.13 mg, respectively (Slit3 LRRD2 corresponds to 10 mg daily). A width of change was confirmed by measuring a bone mineral density for small animals before and after administration, and the results are shown in the following Table 8.

[Table 8]

| Group | Change in BMD (%) | Change in BMC (%) |
|---|---|---|
| Control (n = 12) | $5.27 \pm 1.10$ | $9.51 \pm 2.51$ |
| Slit3 LRRD2 (n = 12) | $6.94 \pm 0.83$ | $10.91 \pm 2.44$ |

(continued)

| Group | Change in BMD (%) | Change in BMC (%) |
|---|---|---|
| HSA-Slit3 LRRD2 fusion protein (LRRD2-3) (n = 11) | 9.03 ± 1.18* | 18.78 ± 2.87* |
| *BMD: bone mineral density, BMC per unit area<br>*BMC: bone mineral content<br>*P < 0.05, vs. non-treated control | | |

As shown in Table 8, albumin-unbound Slit3 LRRD2 has improved BMD and BMC, but their improvement was not statistically significant, and the HSA-Slit3 LRRD2 fusion protein (LRRD2-3) showed effects of significantly improving both BMD and BMC. Therefore, it was confirmed that the HSA-Slit3 LRRD2 fusion protein (LRRD2-3) exhibited a stronger therapeutic effect on bone-related diseases than albumin-unbound Slit3 LRRD2.

[Industrial Applicability]

Since the albumin-bound LRRD2 of the Slit3 protein exhibits the same cytological efficacy as albumin-unbound LRRD2 of the Slit3 protein and has a significantly increased *in vivo* half-life compared to albumin-unbound LRRD2 of the Slit3 protein, bone-related diseases can be more effectively prevented or treated.

The national research and development projects supporting the present invention are as follows.

(1) [National research and development projects supporting the present invention]

[Project Identification Number] 2017-1229 (HI15C0377010017)
[Ministry Name] Ministry of Health and Welfare
[Research Management Agency] Korea Health Industry Development Institute
[Research Project Name] Disease Oriented Translational Research
[Research Title] Discovery of macronuclear cell secretion factors with bone formation promotion
[Contribution Rate] 75/100
[Administrative Organization] Asan Medical Center, Seoul
[Research Period] September 7, 2017 to September 6, 2018

(2) [National research and development projects supporting the present invention]

[Project Identification Number] 2013-2234 (HI13C1634060018)
[Ministry Name] Ministry of Health and Welfare
[Research Management Agency] Korea Health Industry Development Institute
[Research Project Name] Disease Oriented Translational Research
[Research Title] Pharmacokinetic Study of Slit3 LRRD2 and *in vivo* Toxicity Verification Using Slit3 TG Mice
[Contribution Rate] 25/100
[Administrative Organization] Industry & Academic Cooperation in Chungnam National University (IAC)
[Research Period] November 1, 2013 to June 30, 2019

EP 3 932 430 A1

## Claims

1. A pharmaceutical composition comprising albumin-bound LRRD2 of the SLIT3 protein for prevention or treatment of bone-related diseases.

2. The pharmaceutical composition of claim 1, wherein the albumin is human serum albumin.

3. The pharmaceutical composition of claim 2, wherein the human serum albumin is bound to the N-terminus of the LRRD2 of the Slit3 protein.

4. The pharmaceutical composition of claim 3, wherein the human serum albumin comprises an amino acid sequence of SEQ ID NO: 2.

5. The pharmaceutical composition of claim 3, wherein the LRRD2 of the Slit3 protein comprises an amino acid sequence of SEQ ID NO: 3.

6. The pharmaceutical composition of claim 1, further comprising a linker between the albumin and the LRRD2 of the Slit3 protein.

7. The pharmaceutical composition of claim 6, wherein the linker is (GGGGS)n, wherein n is an integer from 1 to 10.

8. The pharmaceutical composition of claim 1, wherein the pharmaceutical composition is administered as an injection.

9. The pharmaceutical composition of claim 1, wherein the bone-related disease is any one or more selected from the group consisting of osteoporosis, fractures, bone loss, osteoarthritis, metastatic bone cancer, and Paget's disease.

[FIG. 1]

# PC DNA 3.1 (+) – SP Cystatin S – HSA(pro-) – H Slit3 LRR D2 – FLAG protein sequence

MARPLCTLLLLMATLAGALADAHKSEVAHRFKDLGEENFKALVLIAFAQYLQQCPFEDHVKLVNEVTEFAKTCVADESAENCDKSLHTLFGDKLCTVATLRETYG
EMADCCAKQEPERNECFLQHKDDNPNLPRLVRPEVDVMCTAFHDNEETFLKKYLYEIARRHPYFYAPELLFFAKRYKAAFTECCQAADKAACLLPKLDELRDE
GKASSAKQRLKCASLQKFGERAFKAWAVARLSQRFPKAEFAEVSKLVTDLTKVHTECCHGDLLECADDRADLAKYICENQDSISSKLKECCEKPLLEKSHCIAE
VENDEMPADLPSLAADFVESKDVCKNYAEAKDVFLGMFLYEYARRHPDYSVVLLLRLAKTYETTLEKCCAAADPHECYAKVFDEFKPLVEEPQNLIKQNCELFE
QLGEYKFQNALLVRYTKKVPQVSTPTLVEVSRNLGKVGSKCCKHPEAKRMPCAEDYLSVVLNQLCVLHEKTPVSDRVTKCCTESLVNRRPCFSALEVDETYVPK
EFNAETFTFHADICTLSEKERQIKKQTALVELVKHKPKATKEQLKAVMDDFAAFVEKCCKADDKETCFAEEGKKLVAASQAALGLISCPSPCTCSNNIVDCRGK
GLMEIPANLPEGIVEIRLEQNSIKAIPAGAFTQYKKLKRIDISKNQISDIAPDAFQGLKSLTSLVLYGNKITEIAKGLFDGLVSLQLLLLNANKINCLRVNTFQDLQNLN
LLSLYDNKLQTISKGLFAPLQSIQTLHLAQNPFVCDCHLKWLADYLQDNPIETSGARCSSPRRLANKRISQIKSKKFRCS **DYKDDDDK\*** **(SEQ ID NO: 9)**

| SP | Cystatin S |
| | |

1 – 20 AA (all 141AA)

| | HSA |
| | |

25 – 609 AA (all 609AA)

| LRR D2 | SLIT3 |
| | |

278 – 486 AA (all 1523AA)

| SP | HSA | LRR D2 |
| | | |

20 AA    585 AA    209 AA    9AA

——— Cystatin S    ——— HSA(pro-)    ——— H Slit3 LRR D2    ——— FLAG    ——— Stop condon

71

[FIG. 2]

[FIG. 3]

[FIG. 4]

[FIG. 5]

[FIG. 6]

[FIG. 7]

<div align="center">

**INTERNATIONAL SEARCH REPORT**

</div>

| International application No. |
|---|
| **PCT/KR2020/002825** |

**A. CLASSIFICATION OF SUBJECT MATTER**

*A61K 47/64(2017.01)i, A61K 38/17(2006.01)i, A61K 9/00(2006.01)i, A61P 19/00(2006.01)i*

According to International Patent Classification (IPC) or to both national classification and IPC

**B. FIELDS SEARCHED**

Minimum documentation searched (classification system followed by classification symbols)
A61K 47/64; A61K 38/16; A61K 39/395; A61K 38/17; A61K 9/00; A61P 19/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: albumin, Slit3, LRR2(leucine-rich repeat domain 2), bone, osteoporosis

**C. DOCUMENTS CONSIDERED TO BE RELEVANT**

| Category* | Citation of document, with indication, where appropriate, of the relevant passages | Relevant to claim No. |
|---|---|---|
| X | KIM, B.-J. et al. Osteoclast-secreted SLIT3 coordinates bone resorption and formation. The Journal of clinical investigation. 2018, vol. 128, no. 4, pages 1429-1441 See abstract; page 1439. | 1-3,9 |
| Y | | 4-8 |
| Y | NCBI. GenBank Accession No. AAA98797.1. albumin [Homo sapiens]. 03 May 1996 See the entire document. | 4 |
| DY | KR 10-1617497 B1 (THE ASAN FOUNDATION) 03 May 2016 See abstract; paragraph [0062]; sequence identifier no. 3; claim 1. | 5,8 |
| Y | KR 10-2019-0003746 A (JANSSEN BIOTECH, INC.) 09 January 2019 See abstract; claims 1, 5, 8-9. | 6-7 |
| Y | NCBI. GenBank Accession No. AAQ89243.1. SLIT3 [Homo sapiens]. 03 October 2003 See the entire document. | 5 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 JUNE 2020 (16.06.2020) | **16 JUNE 2020 (16.06.2020)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2020/002825**

| Patent document cited in search report | Publication date | Patent family member | Publication date |
|---|---|---|---|
| KR 10-1617497 B1 | 03/05/2016 | CN 104582727 A | 29/04/2015 |
| | | CN 104582727 B | 27/04/2018 |
| | | EP 2862580 A1 | 22/04/2015 |
| | | EP 3156074 A1 | 19/04/2017 |
| | | EP 3156074 B1 | 05/06/2019 |
| | | JP 2015-527973 A | 24/09/2015 |
| | | JP 2017-039729 A | 23/02/2017 |
| | | JP 6100367 B2 | 22/03/2017 |
| | | JP 6216849 B2 | 18/10/2017 |
| | | KR 10-2016-0032077 A | 23/03/2016 |
| | | US 2015-0175673 A1 | 25/06/2015 |
| | | US 9802994 B2 | 31/10/2017 |
| | | WO 2013-187730 A1 | 19/12/2013 |
| KR 10-2019-0003746 A | 09/01/2019 | AR 108442 A1 | 22/08/2018 |
| | | AU 2017-263237 A1 | 22/11/2018 |
| | | BR 112018072946 A2 | 19/02/2019 |
| | | CA 3022865 A1 | 16/11/2017 |
| | | CL 2018003148 A1 | 28/12/2018 |
| | | CN 109451726 A | 08/03/2019 |
| | | CO 2018012096 A2 | 22/11/2018 |
| | | CR 20180532 A | 04/03/2019 |
| | | EA 201892561 A1 | 30/04/2019 |
| | | EC SP18083561 A | 30/11/2018 |
| | | EP 3454832 A1 | 20/03/2019 |
| | | JP 2019-523637 A | 29/08/2019 |
| | | MX 2018013784 A | 28/03/2019 |
| | | NI 201800121 A | 18/02/2019 |
| | | PE 20190352 A1 | 07/03/2019 |
| | | SG 11201809700 A | 29/11/2018 |
| | | SV 2018005781 A | 28/03/2019 |
| | | TW 201803892 A | 01/02/2018 |
| | | US 10336812 B2 | 02/07/2019 |
| | | US 2017-0327560 A1 | 16/11/2017 |
| | | US 2019-0292241 A1 | 26/09/2019 |
| | | WO 2017-196647 A1 | 16/11/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101617497

**Non-patent literature cited in the description**

- **H. NEURATH ; R.L.HILL.** The Proteins. Academic Press, 1979
- **MERRIFLELD.** *J. Amer. Chem. Soc.,* 1963, vol. 85, 2149-2156
- **SAMBROOK et al.** Molecular Cloning. Cold Spring Harbour Laboratory Press, 1989
- **BLAUG, SEYMOUR.** Remington's Pharmaceutical Science. Mack Publishing Company, 1975